# EUROPEAN PATENT SPECIFICATION

(11) **EP 3 807 637 B1**
(45) Date of publication and mention of the grant of the patent: **29.07.2026**
(21) Application number: 19733981.5
(22) Date of filing: 13.06.2019
(51) Int. Cl.: G01N 33/50

(54) **A METHOD OF PRECISION CANCER THERAPY**
VERFAHREN ZUR PRÄZISIONSKREBSTHERAPIE
MÉTHODE DE THÉRAPIE ANTICANCÉREUSE DE PRÉCISION

(30) Priority: 13.06.2018 US 201816007146
(43) Date of publication of application: 21.04.2021
(73) Proprietor: King Faisal Specialist Hospital & Research Centre, 11211 Riyadh (SA)
(72) Inventor: ABU KHABAR, Khalid S., Riyadh, 11211 (SA)
(74) Representative: Engelhard, Markus
(86) International application number: PCT/EP2019/065585
(87) International publication number: WO 2019/238873

(56) References cited:
- ROUSSEAU S ET AL: "Inhibition of SAPK2a/p38 prevents hnRNP A0 phosphorylation by MAPKAP-K2 and its interaction with cytokine mRNAs", THE EMBO JOURNAL, EUROPEAN MOLECULAR BIOLOGY ORGANIZATION, vol. 21, no. 23, 2 December 2002 (2002-12-02), pages 6505 - 6514, XP002310599, ISSN: 0261-4189
- ANGELIKA R ET AL: "HLA class II and TNFalpha promotor alleles in IgA deficiency", HUMAN IMMUNOLOGY, NEW YORK, NY, US, vol. 47, no. 1-2, 15 April 1996 (1996-04-15), pages 39, XP027458057, ISSN: 0198-8859, [retrieved on 19960415], DOI: 10.1016/0198-8859(96)84892-0

## Description

### FIELD OF THE INVENTION

The present invention relates to a method of identifying a protein kinase inhibitor for normalizing post-transcriptional regulation as precision cancer therapy.

### BACKGROUND OF THE INVENTION

Cancers are a diverse variety of pathological conditions. One example is breast cancer (BC) which is the most common form of female malignancies, representing a major cause of death from cancer among the women worldwide. Breast cancer is characterized by alteration in the expression of many genes involved in cell cycle, growth and differentiation, DNA repair, apoptosis, inflammation, angiogenesis, invasiveness, and metastasis.

Gene expression is regulated by different mechanisms, including transcriptional, post-transcriptional, and post-translational modification mechanisms. Post-transcriptional control represents an essential level of gene expression fine tuning and comprises processes such as mRNA decay, mRNA transport, and translation. mRNA decay affects the level of available mRNA for translation and it is a tightly regulated process that mainly relies on the presence of a cis-acting sequence in the primary transcript of the mRNA to which *trans-*acting proteins bind and confer stability or instability of the mRNA.

Among the well-known and extensively studied cis-acting mRNA instability determinants are adenylate-uridylate-rich elements (AU-rich elements, AREs). Several ARE binding proteins (ARE-BP) are involved in the pathogenesis of cancer. Specifically, many human tumors are found be associated with deficiency of tristetraprolin (TTP, ZFP36) and/or overexpression of human antigen R (HuR). The aberrant expression of these proteins can derive from misregulation on various regulational levels including transcriptional regulation, epigenetic regulation, post-transcriptional regulation, and post-translational regulation.

Phosphorylation of ARE-BPs by different protein kinases is a mechanism of post-translational modification that highly affects the cellular localization and activity of said ARE-BPs. Protein phosphorylation results in alteration of protein structure and conformation, and modifies its activity and function. The commonly phosphorylated amino acids in eukaryotes are serine, threonine, and tyrosine. The phosphorylation is mediated through the action of a protein kinase (PK), and can be reverse through the action of a phosphatase. Nearly 2% of the human genome encode for PKs, representing about 538 genes which are subdivided into typical, or conventional, and atypical protein kinases, according to the kinase database (http://kinase.com/kinbase/). The majority of typical PKs phosphorylates serine/threonine (STPKs) and only a minority of PKs phosphorylates tyrosine, and atypical PKs are mostly STPKs. To date, FDA has approved 37 small molecule kinase inhibitors and many others are in phase-2/3 clinical trials. Most of the approved kinase drugs are intended for treatment of cancers, and only few of them have been approved for treatment of non-cancerous conditions, such as sirolimus for organ rejection.

Polo-like kinases (PLKs) are a family of regulatory serine/threonine kinases comprising five members including polo-like kinase 1 (PLK-1), as well as PLK-2, PLK-3, PLK-4, and PLK-5. Polo-like kinases are involved in the cell cycle at various stages, including mitosis, spindle formation, cytokinesis, and meiosis. Beyond cell cycle regulation, there is evidence that PLKs play regulatory roles in different cellular pathways and an increasing amount of PLK substrates is revealed. For example, PLK-1 has been found to phosphorylate insulin receptor substrate (IRS), β-catenin, heat-shock protein 70, mTOR, vimentin, and the breast cancer susceptibility protein (BRCA2).

Regulating aberrant expression of cancer-related genes using ARE-BPs is a potential approach for a method of treatment of cancer.

US 2010/0055705 A1 discloses compositions and methods for diagnosing and treating cancer, including TTP as a biomarker and therapeutic option for the treatment of cancer.

EP 2 435 041 B1 relates to a therapeutic combination comprising a PLK-1 inhibitor and an antineoplastic agent.

Bhola et al. [1] disclose a kinome-wide functional screen identifying a role of PLK-1 in acquired hormone-independent growth of ER⁺ human breast cancer.

Maire et al. [2] relates to a PLK-1 inhibitor as potential therapeutic option for the management of patients with triple-negative breast cancer.

Rousseau et al., 2002, THE EMBO JOURNAL, vol. 21, No. 23, page 6505-6514, discloses that phosphorylation of hnRNP Ao can be prevented by SB203580 which is an inhibitor of "stress-activated protein kinase 2a" (SAPK2a/p38). Rousseau et al. does not deal with the identification of any suitable protein kinase inhibitors.

Rousseau et al., 2002, THE EMBO JOURNAL, vol. 21, No. 23, page 6505-6514, discloses that phosphorylation of hnRNP A0 can be prevented by SB203580 which is an inhibitor of "stress-activated protein kinase 2a" (SAPK2a/p38). Rousseau et al. does not deal with the identification of any suitable protein kinase inhibitors.

However, a method of treatment of cancer involving post-transcriptional control of expression of cancer-related genes, comprising administering a protein kinase inhibitor for normalizing the levels of TTP and HuR, has not been described.

The present inventors have used a commercially available kinase inhibitor library that comprises 378 drugs comprising FDA approved agents. High-throughput screening was performed using said library by conducting an optimized highly selective post-transcriptional reporter assay that was designed to identify hits affecting ARE-mediated post-transcriptional regulation. Compounds from the PK inhibitor library were scored as hits if they reduced the expression of ARE-containing reporter activity compared to control reporter activity. The present inventors disclose a method of treatment of cancer using ARE-mediated post-transcriptional regulation of gene expression involving administering a protein kinase inhibitor, namely a B-Raf kinase inhibitor, VEGFR2 inhibitor, or a polo-like kinase inhibitor, preferably a polo-like kinase 1 inhibitor, to a patient in need thereof.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to an in-vitro method of identifying a protein kinase inhibitor for normalizing post-transcriptional regulation in accordance with the appended claims comprising the following steps:
a. transfecting cancer cells or a tissue of a cancer patient with at least one expression vector comprising:
   i. a promoter region comprising a non-inducible constitutively active ribosomal protein gene promoter, preferably a promoter that comprises a modified promoter of the human RPS30 gene that has the nucleic acid sequence of SEQ ID NO: 3 (RPS30M1) or SEQ ID NO:4 (RPS30M-truncated),
   ii. a reporter gene; and
   iii. a 3' untranslated region (3' UTR) containing an AU-rich element, wherein said reporter gene is operably linked to said promoter region and said 3' UTR.
b. providing one or more protein kinase inhibitor(s) to be tested;
c. incubating the cells or a tissue created in step a. with said one or more protein kinase inhibitor(s) to be tested;
d. determining a normalizing effect of said one or more protein kinase inhibitor(s) on post-transcriptional regulation by determining a reporter activity, wherein a reduction in reporter activity indicates that said one or more protein kinase inhibitor(s) is/are suitable for targeted cancer therapy, wherein, preferably, the reduction is a reduction by at least 15 %, preferably by at least 20 %, more preferably by at least 25 %.

In one embodiment, the targeted cancer therapy is a pan-cancer precision oncology therapy capable of treating a cancer regardless of the tissue type or subtype or molecular sub-type of the cancer including but not limited to solid tumors, hematological tumors, leukemias, lymphomas, organ-specific tumors such as breast, colon, prostate, liver, and metastatic tumors of any origin, including subtypes such as hormone positive, hormone negative, Microsatellite Instability high or low, and p53 mutant cancer.

In one embodiment, the method is used as a universal single assay, which is applicable independently of a genetic lesion or type of molecular activity involved with regard to a cancerous disease of said patient.

In one embodiment, in said targeted cancer therapy, a cancer-related gene is post-transcriptionally normalized by administering said protein kinase inhibitor.

In one embodiment, in said targeted cancer therapy, a gene encoding a proinflammatory cytokine is post-transcriptionally normalized by administering said protein kinase inhibitor.

In one embodiment, said administering of said protein kinase inhibitor results in the reduction of expression of a mRNA comprising an AU-rich element.

In one embodiment, said protein kinase inhibitor is selected from inhibitors of kinases of which a kinase activity is aberrant in cancer.

In one embodiment, said one or more protein kinase inhibitor(s) are any of Table 1.

In one embodiment, said more protein kinase inhibitors are a protein kinase inhibitor library.

In one embodiment, said reporter activity is detected by measuring a mRNA level, and/or the expression level of the reporter gene, and/or the activity of the reporter, wherein the expression of said reporter gene is independent of transcriptional induction.

In this aspect, said protein kinase inhibitor, said cancer, said cancer cells, and said patient are as defined below.

### DETAILED DESCRIPTION

The present invention relates to an in-vitro method of identifying a protein kinase inhibitor for normalizing post-transcriptional regulation, as described in the appended claims.

The term "cancer", as used herein, refers to a disease characterized by dysregulated cell proliferation and/or growth. The term comprises benign and malignant cancerous diseases, such as tumors, and may refer to an invasive or non-invasive cancer. The term comprises all types of cancers, including carcinomas, sarcomas, lymphomas, germ cell tumors, and blastomas. In one embodiment, the term cancer relates to breast cancer. In one alternative embodiment, cancer relates to invasive breast cancer, such as triple-negative breast cancer. In one embodiment, a "tumor sample", as used herein, relates to a sample of cancerous tissue of a patient, wherein said sample may derive from a solid or a non-solid cancerous tissue. The tumor sample can be in the form of dissociated cells, aspirations, tissues, tissue slices, or any other form of obtaining tumors or tumor tissues or tumor cells known to the person skilled in the art. In one embodiment, said tumor sample is a sample of breast cancer, such as invasive breast cancer, including triple-negative breast cancer. A control sample or control value is used to estimate the relative expression levels of a gene, such as TTP, HuR, and/or PLK-1 expression, in a diseased organ or tissue compared to a healthy organ or tissue.

The term "invasive breast cancer", as used herein, refers to a breast cancer that spreads beyond the layer of tissue in which it developed into surrounding healthy, normal tissue. Invasive breast cancer may spread from the breast through the blood and lymph system to other parts of the body.

The term "triple-negative breast cancer" or "TNBC", as used herein, refers to a breast cancer that does not express the genes encoding for the estrogen receptor (ER), the progesterone receptor (PR), and HER2/neu.

The term "cancer cell", as used herein, refers to a cell that exhibits abnormal proliferation and divides relentlessly, thereby forming a solid tumor or a non-solid tumor. In some embodiments of the present invention, cancer cell is used synonymously with "pathophysiological cell".

The term "non-cancer cell" or "normal cell", as used herein, refers to a cell which is not affected by aberrant expression and/or abnormal proliferation, and does not derive from cancerous tissue. In some embodiments of the present invention, the terms "normal cell" and "non-cancer cell" are used synonymously with "physiological cell".

A "control sample", as used herein, relates to a sample comprising normal cells for determining normal expression levels in non-cancerous cells. Such a control sample may derive from the patient, wherein said control sample is taken from a healthy tissue, wherein said healthy tissue may derive from the same organ as the tumor sample of the cancerous disease, but a different site not affected by said cancerous disease, or may derive from a different organ not affected by said cancerous disease. A control sample may also relate to a sample of non-cancerous tissue of a healthy individual, or to a sample of a population of healthy individuals. In some embodiments, said control sample(s) may also relate to "control values" which reflect the normal expression levels obtained from analysis of expression in control samples, wherein said control samples derive from healthy tissue of the patient, or healthy tissue of a healthy individual, or healthy tissue of a population of healthy subjects.

The term "cancer-related genes", as used herein, refers to genes that are associated with cancerous disceases, and/or the development of cancerous diseases, and/or metastasis. In one embodiment, aberrant expression of said cancer-related genes promotes formation of a cancerous disease. For example, cancer-related genes include MMP1, MMP13, CXCR4, uPA, uPAR, IL-8, IL-6, NEK2, TOP2A, BIRC5, and SLC2A1. In one embodiment, cancer-related genes refer to proto-oncogenes.

The term "AU-rich element" or "ARE", as used herein, refers to an adenylate-uridylate-rich element in the 3' untranslated region of a mRNA. AREs are a determinant of RNA stability, and often occur in mRNAs of proto-oncogenes, nuclear transcription factors, and cytokines. It contains the core pentamer AUUUA in a UA-rich sequence context of at least an overall 7-nucleotide region. ARE-binding proteins (ARE-BP) bind to AREs and stabilize the mRNA, such as HuR, or destabilize the mRNA, such as TTP.

The term "overexpression", as used herein, refers to an elevated expression level as compared to the expression level in a non-cancer cell, referred to as "normal expression". In some embodiments, expression is compared to normal expression in a control sample, which may derive from healthy tissue of the same individual, wherein said healthy tissue may derive from a different site of the same organ as the cancerous tissue, or from a healthy individual. In some embodiments, expression is compared to normal expression in a healthy subject population. An elevated expression level may also be referred to as "increased expression level". In one embodiment, an elevated expression is an at least two-fold change in expression. The term "decreasing expression", as used herein, relates to decreasing elevated expression levels of overexpressed genes, such as HuR, PLK-1, and/or cancer-related genes, to normalize said overexpression to normal expression. Methods for determining the expression level of a target gene are known to a person skilled in the art, and include northern blot, reverse transcription PCR, real-time PCR, in-situ-hybridization, microarrays, and next generation sequencing.

The term "underexpression", as used herein, refers to a decreased expression level as compared to the expression level in a non-cancer cell, referred to as "normal expression". In some embodiments, expression is compared to normal expression in a control sample, which may derive from healthy tissue of the same individual, wherein said healthy tissue may derive from a different site of the same organ as the cancerous tissue, or from a healthy individual. In some embodiments, expression is compared to normal expression in a healthy subject population. Said decreased expression level may also be referred to as "diminished expression level". The term "enhancing expression", as used herein, relates to increasing decreased expression levels of underexpressed genes, such as TTP, to normalize said underexpression to normal expression.

The term "normal expression" or "normal levels", as used herein, refers to expression levels in non-cancerous cells which are not affected by aberrant expression. In one embodiment, normal expression relates to expression levels of TTP, HuR, PLK-1, and/or other genes, in non-cancerous cells. In one embodiment, normal levels of TTP, HuR, PLK-1, and/or other genes, are assessed in the same subject from which the tumor sample is taken. In one embodiment, normal levels are assessed in a sample from a healthy subject. In one embodiment, normal levels are assessed in a population of healthy individuals.

The terms "normalizing" and "normalizing expression", as used herein, relate to normalizing or restoring expression levels and/or activity of targets, such as TTP, HuR, and/or PLK-1, AU-rich mRNA, and protein products thereof, to healthy, non-cancerous, normal levels, which can be achieved by administering an effective dose of a protein kinase inhibitor to a patient in need thereof having abnormal expression of TTP, HuR, and/or PLK, and/or increased activity of AU-rich element-mediated pathways. In one embodiment, said protein kinase inhibitor is a B-Raf kinase inhibitor, VEGFR2 inhibitor, or polo-like kinase inhibitor, for example a polo-like kinase 1 inhibitor. In one embodiment, normalizing expression may relate to increasing TTP expression and/or reducing HuR expression. In one embodiment, said increase in TTP expression and/or reduction in HuR expression results in downregulation of expression of cancer-related genes. In one embodiment, when referring to "normalizing post-transcriptional regulation", it is meant that the level of post-transcriptional regulation in a cancer cell adjusts to a level of post-transcriptional regulation that is present in a non-cancerous cell, preferably by treatment with a protein kinase inhibitor. In one embodiment, a "normalizing effect" refers to an effect, preferably an effect of a protein kinase inhibitor, which induces a normalization of abnormal post-transcriptional regulation of AU-rich mRNAs in cancer cells towards the post-transcriptional regulation and/or expression levels typically found in non-cancerous cells. In one embodiment, an "aberrant" expression and an "aberrant" activity mean expression and activity that deviate from "normal" expression and "normal" activity in an individual not suffering from cancer, respectively.

The term "TTP" or "tristetraprolin", as used herein, refers to a protein which binds to AU-rich elements (AREs) in the 3'-untranslated regions of ARE-containing mRNAs, and promotes degradation of said mRNAs. TTP is also known as zinc finger protein 36 homolog (ZFP36). In one embodiment, interactions of TTP and target mRNAs are affected by the phosphorylation state of TTP.

The term "HuR" or "human antigen R", as used herein, refers to a protein containing RNA-binding domains which binds to cis-acting AU-rich elements (AREs) of mRNA. Binding of HuR to an ARE of an mRNA stabilizes said mRNA. HuR post-translationally regulates gene expression by binding to and stabilizing ARE-containing mRNA. HuR levels may be elevated in cancer cells thereby increasing mRNA stability of cancer-related genes.

The term "TTP/HuR ratio", as used herein, relates to the ratio of expression levels of TTP to HuR. In one embodiment, said expression levels relate to mRNA or protein. In one embodiment, said TTP/HuR ratio is a biomarker of invasiveness or metastatic potential, i.e. the aggressiveness of cancer, in particular breast cancer. In one embodiment, a low TTP/HuR ratio indicates invasive/metastatic cancer, a high TTP/HuR ratio indicates a healthy individual or a patient with non-invasive/non-metastatic cancer. In one embodiment, a method of treatment according to the present invention is used to treat or prevent invasiveness or metastasis of cancer by increasing ("normalizing") the TTP/HuR ratio.

The term "protein kinase", as used herein, refers to an enzyme capable of phosphorylating other proteins by transferring a phosphate group from a nucleoside triphosphate to amino acids of proteins, such as serine and threonine, and/or tyrosine. Phosphorylation of proteins may result in functional modification of said proteins by changing cellular location, activity, and/or associated with other proteins. In one embodiment, a protein kinase may relate to a serine/threonine-specific protein kinase or a tyrosine-specific protein kinase. A list of examples for kinase inhibitors are given in Table 1.

The term "inhibitor", as used herein, refers to an enzyme inhibitor or receptor inhibitor which is a molecule that binds to an enzyme or receptor, and decreases and/or blocks its activity. The term may relate to a reversible or an irreversible inhibitor.

The term "protein kinase inhibitor", as used herein, refers to an inhibitor that blocks the action of one or more protein kinases. In one embodiment, said term relates to an inhibitor that attenuates the action of one or more protein kinases. In one embodiment, said protein kinase inhibitor is a serine/threonine protein kinase inhibitor, such as a B-Raf kinase inhibitor or a polo-like kinase inhibitor, or a tyrosine kinase inhibitor, for example a VEGFR2 inhibitor.

The term "PLK" or "polo-like kinase", as used herein, refers to a family of regulatory serine/threonine kinases of the cell cycle which plays a role in mitosis, spindle formation, meiosis, and cytokinesis. Polo-like kinase is a family of regulatory serine/threonine kinases that comprise five members including PLK-1, PLK-2, PLK-3, PLK-4, and PLK-5. They are involved in the cell cycle at different levels including mitosis, spindle formation, cytokinesis, and meiosis. PLKs share a conserved catalytic serine/threonine kinase domain at the N-terminus and a C-terminal containing two motifs called polo-boxes (polobox domain or PBD) that are involved in PLK localization, activation and binding to substrates. The N-terminals containing kinase domains are very highly conserved among all members of the family while the C-terminal carrying two polo-boxes is much less conserved among them. The N- and C domains are joining by a linker region known as the polo-box cap (Pc) that represents a part of the PBD. The PLKs are activated by upstream kinases through phosphorylation of PLK catalytic kinase domain at a short region called T-loop (containing Thr210). It has been reported that PLK-1 is phosphorylated by polo-like kinase kinase 1 (PLKK1) and protein kinase A. Also, Aurora A phosphorylates PLK-1 at Thr210 by the aid of *bora* which induces a conformation of PLK-1 priming it for Aurora-induced phosphorylation. In addition, binding of phosphorylated docking proteins to PBD leads to PLK activation. Phosphorylation of a substrate by other kinases (such as Cdk1 or Cdk5) is required to turn on PLKs activity. Absence of these phosphorylated proteins make the PBD interacts with the catalytic domain and thus inactivate PLK. Binding of phosphopeptides to PBD results in the release of the catalytic domain which converts PLK to its active form. Finally, the activity of PLKs is abolished by proteolytic degradation through the ubiquitin-proteasome pathway by the action of ubiquitin-ligase Anaphase Promoting Complex (APC) as cells exit mitosis.

The term "PLK-1" or "polo-like kinase 1", as used herein, refers to a specific kinase being a member of the family of polo-like kinases. PLK-1 is considered to be a proto-oncogene as it may be overexpressed in tumor cells. In humans, PLK-1 is expressed in the late interphase (G2) and M phases (prophase, metaphase and anaphase). During interphase and prophase, PLK-1 localizes to centrosomes, whereas at metaphase it binds to spindle poles. In the anaphase, it is distributed to the central spindle while during cytokinesis it is found in the midbody. Entry into mitosis is controlled by PLK-1, an action that is attributed to regulation of the activity of Cdk1-cyclin-B. The latter is a master regulator of M phase that is activated during G2/M transition by its dephosphorylation at ATP-binding site secondary to the action of cell division cycle25 phosphatase (Cdc25). Furthermore, chromosome segregation during anaphase and exit from mitosis are also regulated by PLK-1. This action is mediated through phosphorylation of the APC/Cyclosome (APC/C) ubiquitin ligase.

The term "PLK-1 inhibitor", as used herein, refers to an inhibitor of polo-like kinase 1. In one embodiment, said PLK-1 inhibitor is specific, i.e. said PLK-1 inhibitor only inhibits PLK-1 and does not inhibit other PLKs, such as PLK-2, PLK-3, PLK-4, and/or PLK-5, at nanomolecular concentrations. For example, volasertib is a specific PLK-1 inhibitor. In another embodiment, said term may relate to a PLK-1 inhibitor that binds to PLK-1 and that also binds to other proteins, such as other PLKs, wherein said PLK-1 inhibitor has a lower binding affinity to other proteins than to PLK-1. For example, BI-2536 is a non-specific PLK-1 inhibitor, which binds to PLK-1, and also binds to PLK2 and PLK3 at nanomolecular concentrations.

The term "administering", as used herein, refers to intravenous, oral, nasal, mucosal, intrabronchial, intrapulmonary, intradermal, subcutaneous, intramuscular, intravascular, intrathecal, intraocular, intraarticular, intranodal, intratumoral, or intrametastatical administration of a protein kinase inhibitor to a patient in need thereof. In one embodiment, the term "administering" may also relate to incubating a cell or tissue with a compound such as a protein kinase inhibitor.

The term "co-administering", as used herein, refers to combined administration of a protein kinase inhibitor with at least another substance, such as a protein kinase inhibitor selected from the group of protein kinase inhibitors that is active (reduces reporter activity) in the disclosed post-transcriptional reporter assay using the tissues or cells of a patient. In other words, targeting more than one aberrant pathway, by co-administering at least one other substance, can be beneficial to the patients.

The term "co-administering", as used herein, also refers to combined administration of a protein kinase inhibitor with one or more other substances, such as a chemotherapeutic agent, a checkpoint inhibitor, and/or IFN to a patient in need thereof.

The term "effective dose", as used herein, refers to a dose of a drug, such as a protein kinase inhibitor, which is in the range between the dose sufficient to evoke a therapeutic effect and the maximum tolerated dose. In one embodiment, a method of treatment of cancer according to the present invention comprises administering an effective dose of a protein kinase inhibitor, preferably a polo-like kinase 1 inhibitor, to a patient in need thereof. In one embodiment, a method of treatment of cancer according to the present invention comprises administering an effective dose of a protein kinase inhibitor, preferably a polo-like kinase 1 inhibitor, to a patient in need thereof, wherein said effective dose is in a dose range established for a different method of treatment comprising administering said protein kinase inhibitor, preferably a polo-like kinase 1 inhibitor, wherein said different method of treatment is for a disease, which is not characterized by one of the following: underexpression of tristetraprolin (TTP), overexpression of human antigen R (HuR), overexpression of polo-like kinase 1 (PLK-1), underexpression of TTP and overexpression of HuR, underexpression of TTP and overexpression of PLK-1, overexpression of HuR and overexpression of PLK-1, underexpression of TTP and overexpression of HuR and overexpression of PLK-1, in pathophysiological cells compared to expression in physiological cells. In one embodiment, said protein kinase inhibitor is volasertib, and said effective dose is in the range of 150 mg to 300 mg once per day to once per week.

The term "patient", as used herein, refers to a human or an animal having a cancer which is characterized by one of the following: underexpression of TTP and overexpression of HuR, underexpression of TTP and overexpression of PLK-1, overexpression of HuR and overexpression of PLK-1, underexpression of TTP and overexpression of HuR and overexpression of PLK-1, increased AU-rich element-mediated post-transcriptional activity, in cancer cells compared to expression in normal cells. The terms "subject" and "individual", as used herein, are used synonymously, and relate to a human or an animal.

The term "chemotherapeutic agent", as used herein, refers to a cytotoxic agent which is of use in chemotherapy of cancer. For example, a chemotherapeutic agent may relate to an alkylating agent, such as cyclophosphamide, mechlorethamine, chlorambucil, melphalan, dacarbazine, nitrosoureas, and temozolomide, or to an anthracycline, such as daunorubicin, doxorubicin, epirubicin, idarubicin, mitoxantrone, valrubicin, or to a cytoskeletal disruptor, such as paclitaxel, docetaxel, abraxane, and taxotere, or to an epothilone, or to a histone deacetylase inhibitor, such as vorinostat and romidepsin, or to an inhibitor of topoisomerase I, such as irinotecan and topotecan, or to an inhibitor of topoisomerase II, such as etoposide, teniposide, and tafluposide, or to a kinase inhibitor, such as bortezomib, erlotinib, gefitinib, imatinib, vemurafenib, and vismodegib, or to a nucleotide analogue, such as azacitidine, azathioprine, capecitabine, cytarabine, doxifluridine, fluorouracil, gemcitabine, hydroxyurea, mercaptopurine, methotrexate, and tioguanine, or to a peptide antibiotics, such as bleomycin and actinomycin, or to a platinum-based agent, such as carboplatin, cisplatin, and oxaliplatin, or to a retinoid, such as tretinoin, alitretinoin, and bexarotene, or to a vinca alkaloid derivative, such as vinblastine, vincristine, vindesine, and vinorelbine. In one embodiment, in a method of treatment of cancer according to the present invention, a chemotherapeutic agent is co-administered with said protein kinase inhibitor, wherein preferably, said chemotherapeutic agent is commonly used for the same type of cancer.

The term "checkpoint inhibitor", as used herein, refers to an agent used in cancer immunotherapy. A checkpoint inhibitor blocks an inhibitory immune checkpoint and thus restores immune system function, for example, an inhibitor of the immune checkpoint molecule CTLA-4, such as ipilimumab, or an inhibitor of PD-1, such as nivolumab or pembrolizumab, or an inhibitor of PD-L1, such as atezolizumab, avelumab, and durvalumab. In many of the embodiments, a checkpoint inhibitor relates to an antibody which targets a molecule involved in an immune checkpoint.

The term "interferon", or "IFN", as used herein, refers to a group of cytokines which are used for communication between cells and which trigger the immune system. Interferons comprise three classes which are Type-I interferons, Type-II interferons, and Type-III interferons. In one embodiment, said protein kinase inhibitor is co-administered with a Type-I, Type-II or Type-III IFN.

The term "Type-I IFN", as used herein, relates to a large subgroup of interferons comprising IFN-α, IFN-β, IFN-ε, IFN-κ, IFN-τ, IFN-ζ, and IFN-ω.

The term "Type-II IFN", as used herein, relates to IFN-y.

The term "Type-III IFN", as used herein, relates to IFN-λ1, 2, 3, and 4.

The term "IFNy", or "interferon gamma", as used herein, refers to a cytokine which is the only member of the type II class of interferons, and is an important activator of macrophages. Aberrant expression of IFNγ is associated with autoinflammatory and autoimmune diseases. IFNγ has antiviral, immunoregulatory and anti-tumor properties.

The term "post-transcriptional control" or "post-transcriptional regulation", as used herein, refers to the control of gene expression at the RNA level including translation of the mRNA. The stability and distribution of different transcripts may be regulated by RNA binding proteins that control processes such as alternative splicing, nuclear degradation, processing, nuclear export, sequestration, and translation.

The terms "targeted cancer therapy" and "precision cancer therapy", as used herein, relate to the prevention or treatment of a cancer in a patient by administering an effective amount of a therapeutic agent to said patient. Preferably, prior to administering said therapeutic agent, it is tested whether the patient is likely to respond to said therapeutic agent by means of a method of identifying a protein kinase inhibitor according to the present invention. It is also called Precision Oncology and Precision Medicine. Said cancer therapy is "targeted" (and thus "precise") since, prior to said therapy, it is determined which therapeutic agent, namely which protein kinase inhibitor, is able to reduce reporter activity in a cancer cell of said patient comprising a reporter system (vector), and said reduced reporter activity is an indicator that the cancer/cancer cells of said patient will respond to said protein kinase inhibitor. Accordingly, a suitable protein kinase inhibitor for treating said patient can be chosen using a method of identifying a protein kinase inhibitor for normalizing post-transcriptional regulation. A method of identifying a protein kinase inhibitor for normalizing post-transcriptional regulation is a tool for precision oncology allowing for determining a suitable protein kinase inhibitor for treating a cancer patient. Said tool for precision oncology may comprise i) obtaining cells from a patient, for example from a solid tumors or lymph node or metastatic site by biopsy or aspiration, or from a blood tumor by obtaining blood cells, ii) subjection the cells to the post-transcriptional reporter expression vector for transfection and/or transduction, iii) treating the vector-transfected or transduced cells to at least one protein kinase inhibitor, preferably to a protein kinase inhibitor library, iv) determining at least one protein kinase inhibitor that reduces the reporter activity, preferably by at least 15%, 20%, 25%, 50%, or more than 50%, more preferably by at least 90 %, v) administering at least one protein kinase inhibitor determined in step iv) alone or in combination with another protein kinase inhibitor determined in step iv), and/or in combination with a chemotherapeutic agent or any other therapeutic agent, to a patient in need thereof.

The term "transfecting", as used herein, relates to transferring an expression vector, preferably comprising a reporter system, to a target cell. Methods for transiently or stably transfecting a target cell with DNA/vectors are well known in the art. These include, but are not limited to electroporation, calcium phosphate co-precipitation, cationic polymer transfection, lipofection, viral transfection, and microinjection of an expression vector. In one embodiment, the term "transfecting" may also relate to "transducing", which largely refer to either infection or transduction of viral-based vectors including but not limited to lentiviral vectors, adenovirus vectors, pseudovectors, adenovirus associated virus vectors. Alternatively, the reporter mRNA can also be introduced by any of these methods. In one embodiment, transfection of cancer cells or tissue of a cancer patient with an expression vector results in the creation of cell lines harboring the expression vector and/or results in cells/tissue transiently expressing the vector encoded genetic information for at least a few hours, such as at least 1 hour, preferably at least 3 hours.

The terms "expression vector" and "vector", as used herein, relate to an expression system which comprises a reporter gene. Expression vectors are common tools to study the biological function of a gene/protein, and various types (e.g. plasmid-based or viral-based vectors) are known in the art. The use of an expression vector allows for the identification of compounds that affect post-transcriptional regulation of genes/reporter. An expression vector used in the present invention may be in any form, such as a plasmid, nucleic acid, vector, lentivirus vector, or adeno vector. A vector used in the present invention comprises at least a promoter region comprising a ribosomal protein gene promoter, preferably a modified RPS30 gene (RPS30M1) or a fragment thereof, a reporter gene, and a 3' untranslated region containing an AU-rich element. An expression vector used in the invention allows for a selective assessment of post-transcriptional events, such as in response to potential drug candidates, particularly in response to a protein kinase inhibitor. In one embodiment, an expression vector may additionally comprise a selectable marker. For the generation of stable cell lines, clones can be selected using various selectable markers, which include, but are not limited to neomycin, blasticidin, puromycin, zeocin, hygromycin, and dihydrofolate reductase (dhfr).

The term "promoter", as used herein, relates to a region of DNA that initiates transcription of a particular gene. An expression vector used in the present invention comprises a promoter derived from ribosomal protein being transcriptionally non-inducible and constitutively active. In one embodiment, promoters such as cellular promoters that lack inducible transcriptional elements can be used. In one embodiment, a promoter used in the present invention is a promoter derived from a ribosomal protein being transcriptionally non-inducible and constitutively active, such as RPS30 or RPS23. An expression vector used in the present invention preferably comprises a promoter derived from ribosomal protein S30 (RPS30) being transcriptionally non-inducible and constitutively active. In one embodiment, a promoter used in the present invention comprises a promoter of the human RPS30 gene, preferably a modified promoter of the human RPS30 gene that has the sequence of SEQ ID NO: 3 which is modified ribosomal protein promoter 30 (RPS30M1) or SEQ ID NO:4 (RPS30M-truncated).

The term "reporter gene", as used herein, relates to a gene used to study post-transcriptional regulation, such as a gene encoding luciferase or nanoluciferase. In one embodiment, said reporter gene has preferably been modified by reducing the presence of UU/UA dinucleotides within the sequence of said reporter gene.

The terms "3' UTR" and "3' untranslated region" generally refers to a section of messenger RNA (mRNA) that immediately follows a translation termination codon. Regulatory regions within the 3'-untranslated region can influence polyadenylation, translation efficiency, localization, and stability of the mRNA. 3' UTR may comprise regulatory regions such as AU-rich elements.

The term "reporter activity", as used herein, relates to activity levels of a reporter gene, such as luciferase activity. Reporter activity can be measured by means known to a person skilled in the art and depends on the reporter gene used. In one embodiment, the mRNA levels and/or expression of said reporter gene are measured to determine reporter activity, for example by means of real time RT-PCR, Northern blots, RNase protection assays, or any other mRNA or RNA detection method. Alternatively, protein levels can be measured, e.g. when secreted using ELISA or Western blotting. Alternatively, fluorescence and chemoluminescence from reporters, such as GFP or luciferase, respectively, can also be measured. In one embodiment, said reporter encodes an enzyme and/or a fluorophore, and said activity is measured by detecting emitted light, color, and/or fluorescence. In one embodiment, said reporter relates to luciferase and a luciferase activity level is quantified by a luminometer.

When referring to a "reduction in reporter activity", it is meant that the activity and/or expression of the reporter gene is reduced in a treated cell compared to a control cell, said reduction for example occurring upon treatment with a compound such as a protein kinase inhibitor. In one embodiment, a reduction in reporter activity upon treatment with a protein kinase inhibitor indicates that said protein kinase inhibitor it suitable for using said protein kinase inhibitor in a method of treatment of cancer in a patient and/or for using said protein kinase inhibitor in a method of post-transcriptional control of cancer-related genes. In one embodiment, said the reporter activity of a cell treated with a protein kinase inhibitor is reduced by at least 10%, by at least 15%, by at least 20%, or by at least 25% upon treatment with said protein kinase inhibitor, preferably reduced by at least 15 %, preferably by at least 20 %, more preferably by at least 25 %, most preferably by at least 90 %. In one embodiment, a "treated cell" is a cell treated with a protein kinase inhibitor.

The term "suitable for targeted cancer therapy", as used herein, relates to a suitability of a protein kinase inhibitor for using said protein kinase inhibitor in a method of treatment of cancer in a patient and/or for using said protein kinase inhibitor in a method of post-transcriptional control of cancer-related genes. In one embodiment, a protein kinase inhibitor that is "suitable" is capable of reducing reporter activity in a method of identifying a protein kinase inhibitor for normalizing post-transcriptional regulation.

The term "UU/UA dinucleotide" or "UU/UA coding dinucleotide", as used herein, relates to an RNase L cleavage site which comprises a uracil-uracil or a uracil-adenine dinucleotide. When referring to a reporter gene or reporter coding region being "with reduced UU/UA" or "reduced from UU/UA", it is meant that a codon comprising a UU and/or UA dinucleotide has been exchanged for an alternative codon not comprising a UU and/or UA dinucleotide, wherein said codon and said alternative codon code for the same amino acid, or it is meant that at least one codon of an adjacent pair of codons comprising a UU and/or UA dinucleotide has been exchanged for an alternative codon coding for the same amino acid so that said adjacent pair of codons does no longer comprise a UU and/or UA dinucleotide.

In one embodiment, the selected protein kinase inhibitor using the said post-transcriptional assay has an additional benefit of being also cytokine inhibitor and thus can further benefit the patients receiving therapy from cytokine-related inflammatory response. Inflammatory cytokine response can happen during treatment of cancer patients with certain therapeutics such as monoclonal antibodies.

In one embodiment, important features of the "precision oncology assay" of the present invention, namely the method of identifying a protein kinase inhibitor for normalizing post-transcriptional regulation as precision cancer therapy, are:
1. Independent on tumor type or tissue type.
2. Independent on genetic lesion, where genetic lesion means: mutations, single nucleotide polymorphism, copy number, amplification, deletion, fusion, mismatch repair defect, microsatellite instability, chromosomal abnormality.
3. Independent on molecular activity, where molecular activity means signaling aberrations, over-expression, constitutive activation of receptor activity, aberrant kinase activity, etc.
4. Single assay as opposed to individual assay for each genetic or molecular lesion.
5. Actionable approach, i.e., the patient's specific cancer can be treated with one or more of the kinase inhibitor drug selected from the screen. Thus the suitable protein kinase inhibitor is identified prior to treatment of the patient.
6. Ability to reduce inflammatory cytokine and cytokine-related disease that are associated with cancer therapeutics.

The term "universal single assay", as used herein, relates to an assay which can be ubiquitously applied in the context of various cancerous diseases, and can be used independently of the genetic lesion or the type of molecular activity involved with regard to the disease. In one embodiment, a universal single assay is broadly applicable to various diseases in contrast to an assay that is specific with regard to a feature of a certain disease, such as an involved kinase, genotype, mutation, microsatellite instability, mismatch repair, and/or copy amplification. In one embodiment, a universal single assay is not specific to a certain cancer type, but is useful for various types of cancerous diseases, and is thus a pan-cancer precision oncology approach.

### BRIEF DESCRIPTION OF THE FIGURES

**Figure 1****:** Kinome inhibitors screening on post-transcriptional gene regulation.
   (A) 387 Kinase inhibitors screen scatter plot: AU-rich element luciferase reporter activity absolute values are plotted on the y axis against 378 corresponding kinase inhibitors on the x axis using a primary screening leading to 87 (A) and secondary screening (B).
**Figure 2****:** The post-transcriptional precision cancer assay leads to 14 inhibitors.
   Luciferase activity in MDA MB231 cell line treated with DMSO (control) and several inhibitors from the screen. Columns, mean value of experiments done in triplicate; bars, standard error of the mean (SEM), normalized luciferase activity (fold) relates to ARE/non-ARE ratio. The bottom show the inhibitors and the targeted kinase pathway.

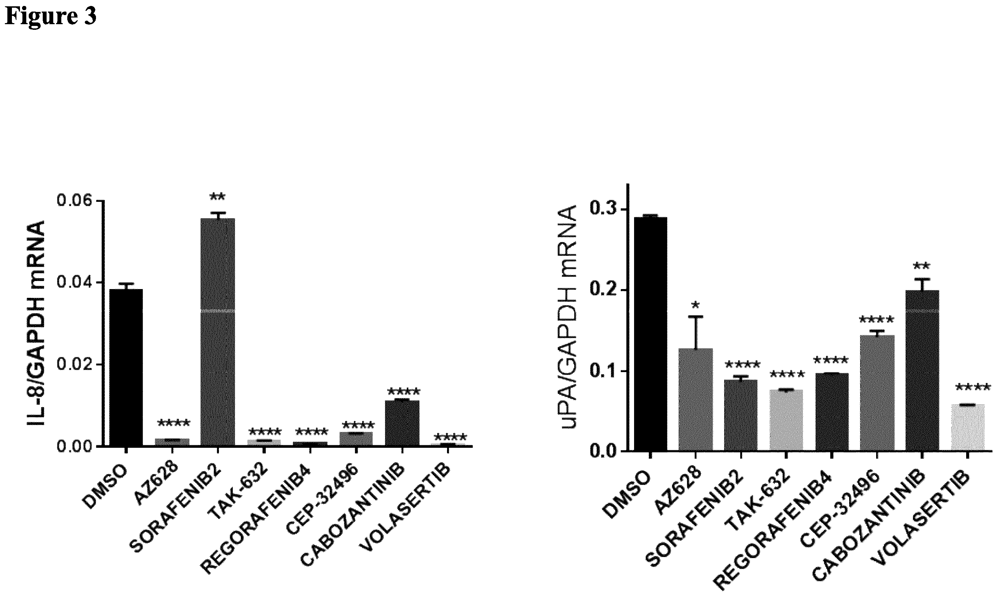

**Figure 3****:** The effect of different kinase inhibitors on AU-rich mRNA levels. DMOS-treated readings are control. Values are shown as mean ± SEM and statistical analysis was performed using Student's t-test.

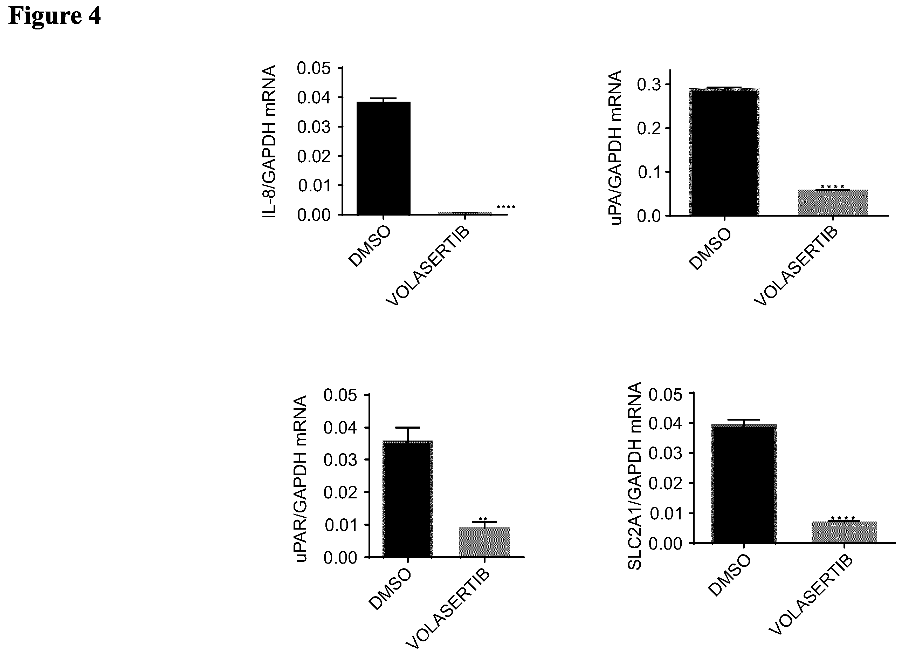

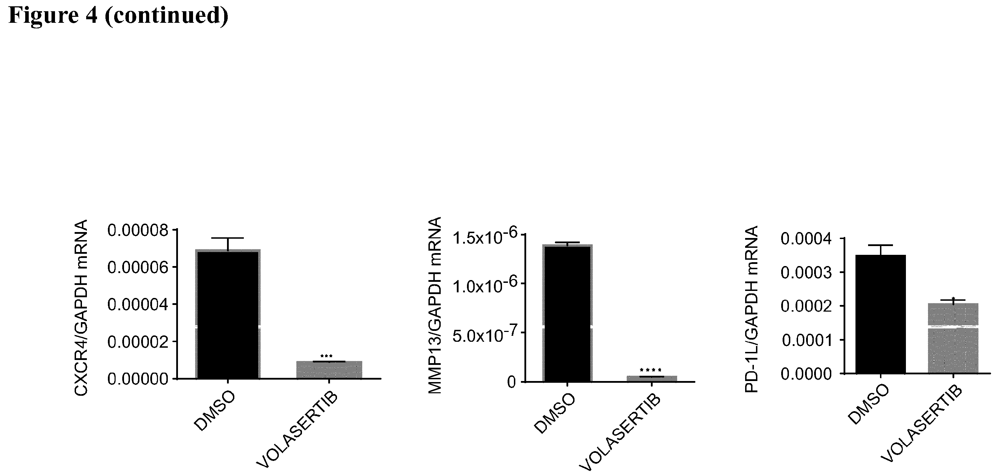

**Figure 4****:** Effect of an example of protein kinase inhibitor, volasertib, which is PLK1 inhibitor on the expression of various cancer-related genes having ARE-containing mRNAs. mRNA expression of IL-8, uPA, uPAR, SLC2A1, CXCR4, MMP13 is shown for control cells and volasertib treated MDA-MB-231 cells. Values are shown as mean ± SEM and statistical analysis was performed using Student's t-test.

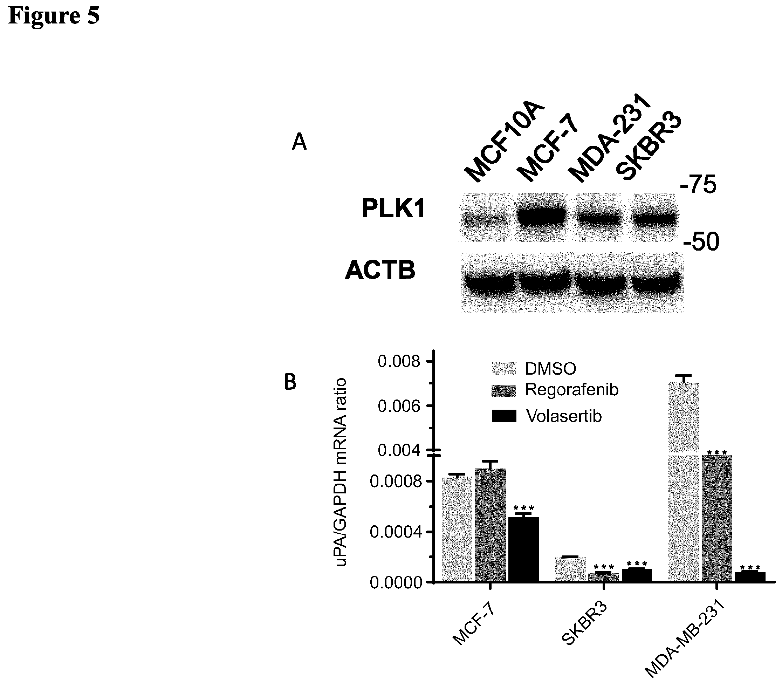

**Figure 5****:** Example of protein kinase inhibitor effective in three different cell lines that over-express the kinase. (A) The PLK1 kinase expression is higher in tumor cells when compared to normal cells. (B) Effect of two kinase inhibitors, PLK1 inhibitor (volasertib) and raf-1 inhbitor.

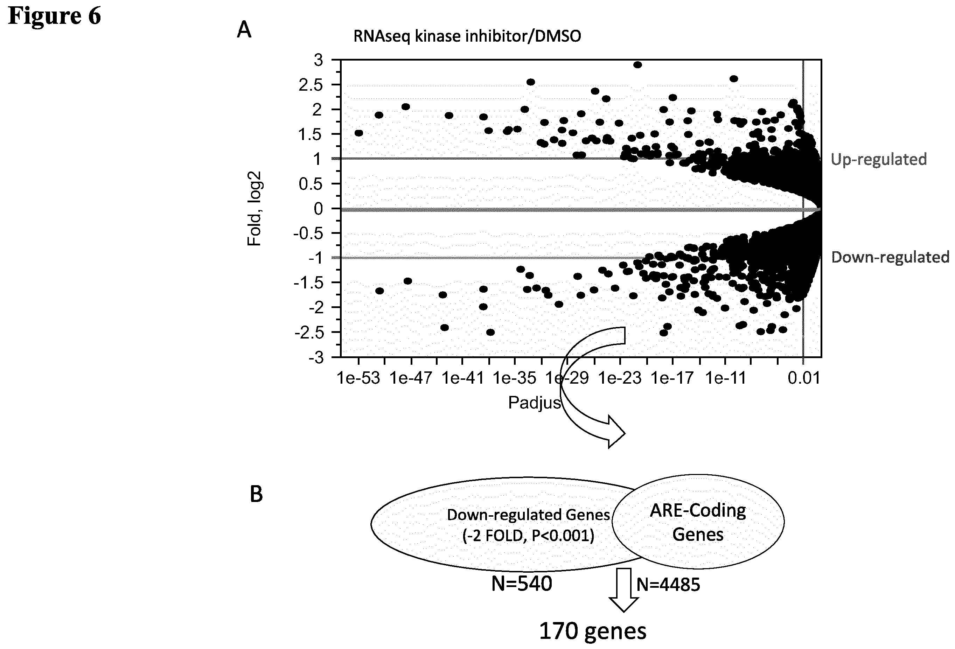

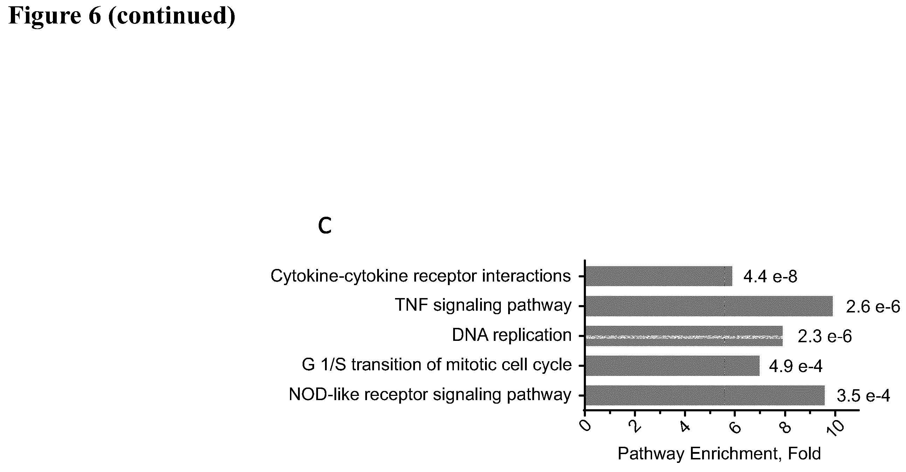

**Figure 6****:** Pathways most affected by the protein kinase inhibitor in MDA-MB-231cells as explored by RNAseq analysis.

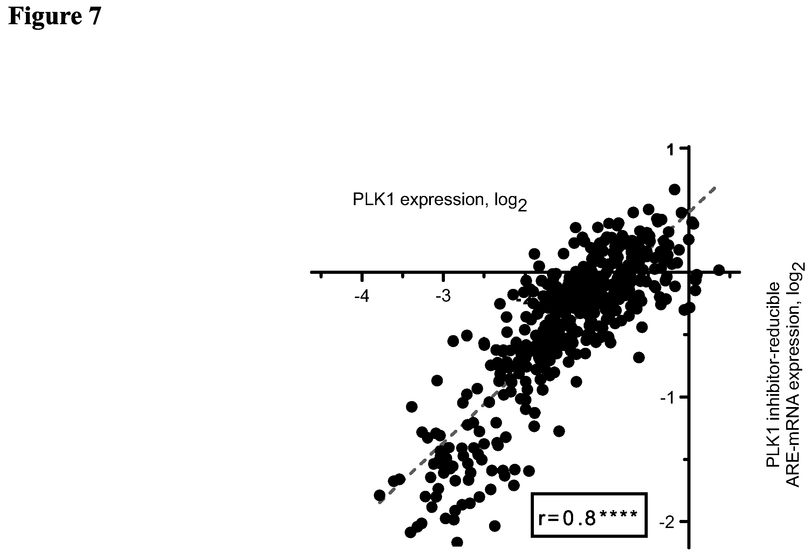

**Figure 7****:** Correlation between the mRNA levels of PLK1, an example of kinases, and expression of AU-rich mRNAs that are also can be reduced by a PLK1 kinase inhibitor.

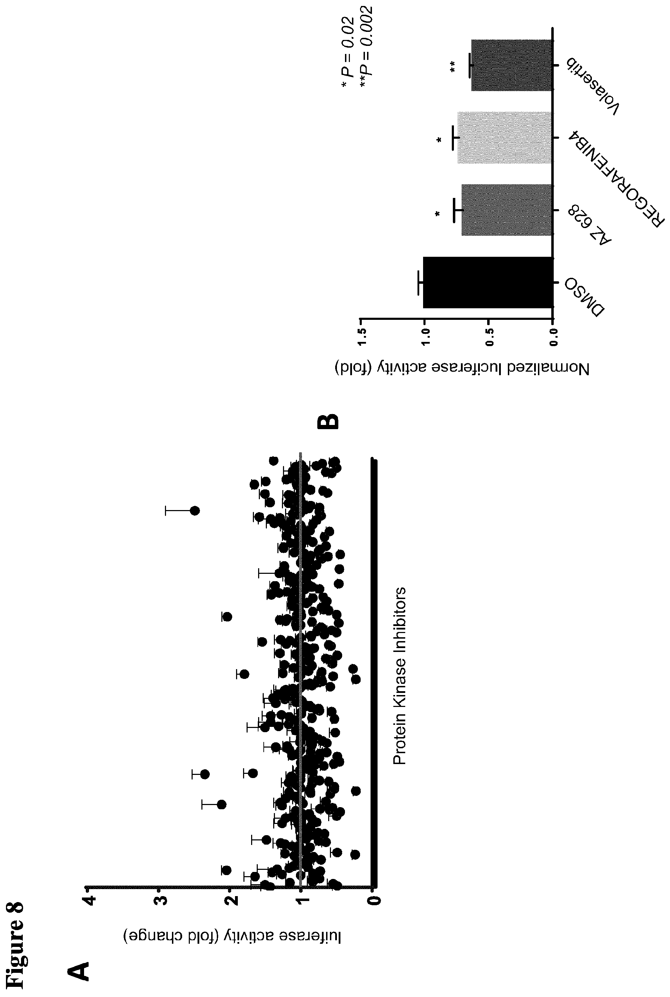

**Figure 8****:** Kinome inhibitors screening on post-transcriptional gene regulation.
   (A) Kinase inhibitor screen scatter plot: TNF-α-ARE luciferase reporter activity absolute values are plotted on the y axis against 378 corresponding kinase inhibitors on the x axis.
   (B) Luciferase activity in MDA MB231 cell line treated with DMSO (control), AZ 628, Regorafenib, and Volasertib for 24 h. Columns, mean value of experiments done in triplicate; bars, standard error of the mean (SEM), normalized luciferase activity (fold) relates to ARE/non-ARE ratio.

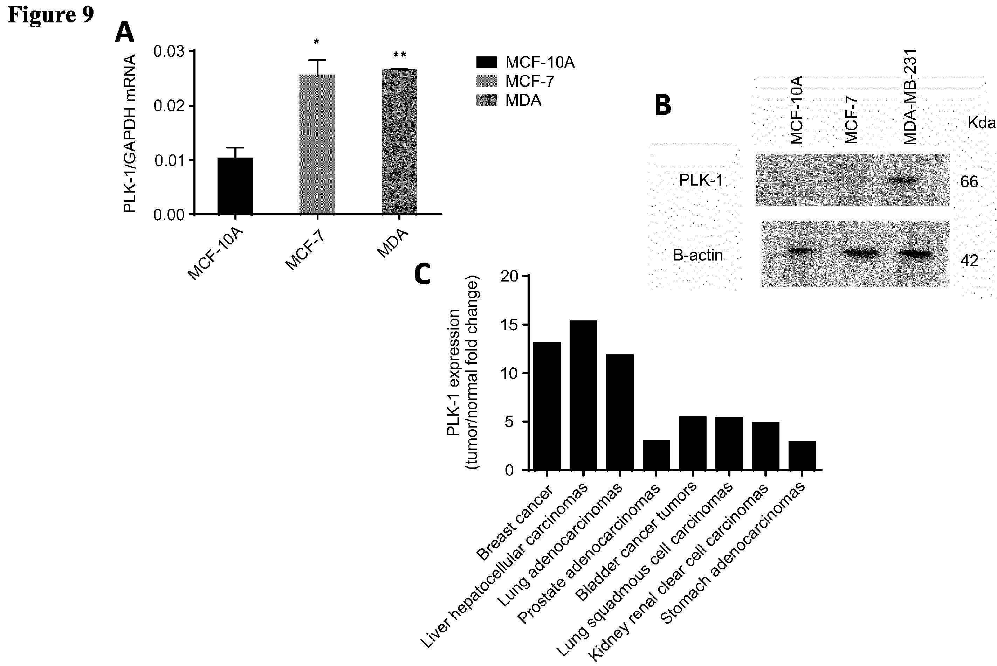

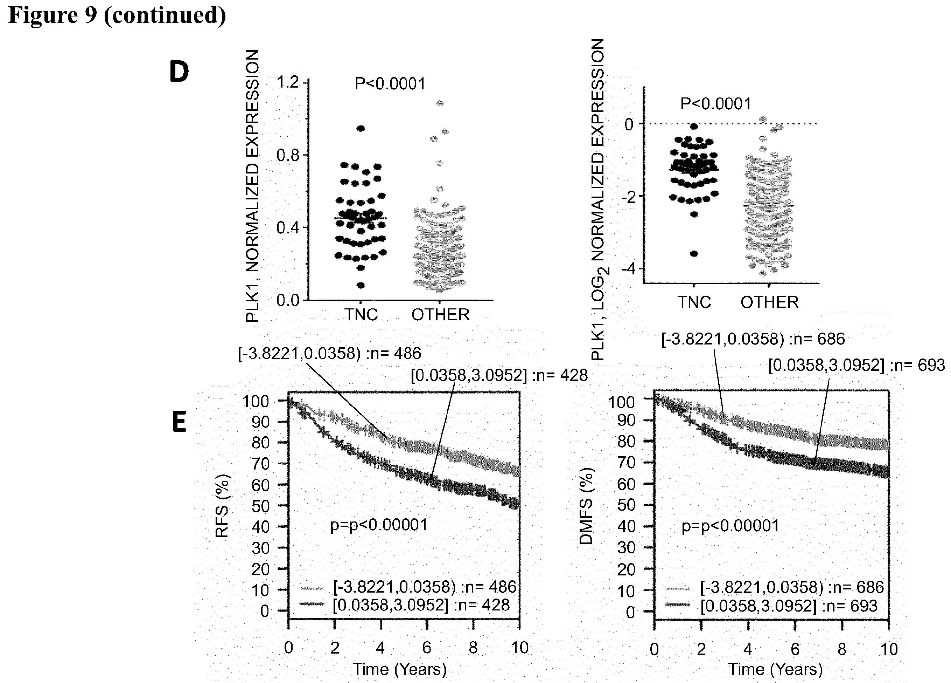

**Figure 9****:** Expression of PLK-1 in normal cells and cancer cells.
   (A) PLK-1 mRNA expression in normal cells and breast cancer cell lines quantified by RT-PCR using FAM-labelled PLK-1 and a VIC-labelled GAPDH probe.
   (B) PLK-1 protein expression in normal cells and breast cancer cells using primary antibodies for PLK-1 and beta-actin (control).
   (C) PLK-1 expression (mRNA tumor/normal fold change) in different types of cancer.
   (D) PLK-1 expression in triple negative cancer (TNC) compared to other types of breast cancer.
   (E) The effect of PLK-1 overexpression on the 10-years relapse-free survival (RFS) and distant metastasis-free survival (DMFS) of cancer patients. Values are shown as mean ± standard error of the mean (SEM), and comparison was performed using Student's t-test, wherein *P ≥ 0.01, ***P* ≥ 0.001.

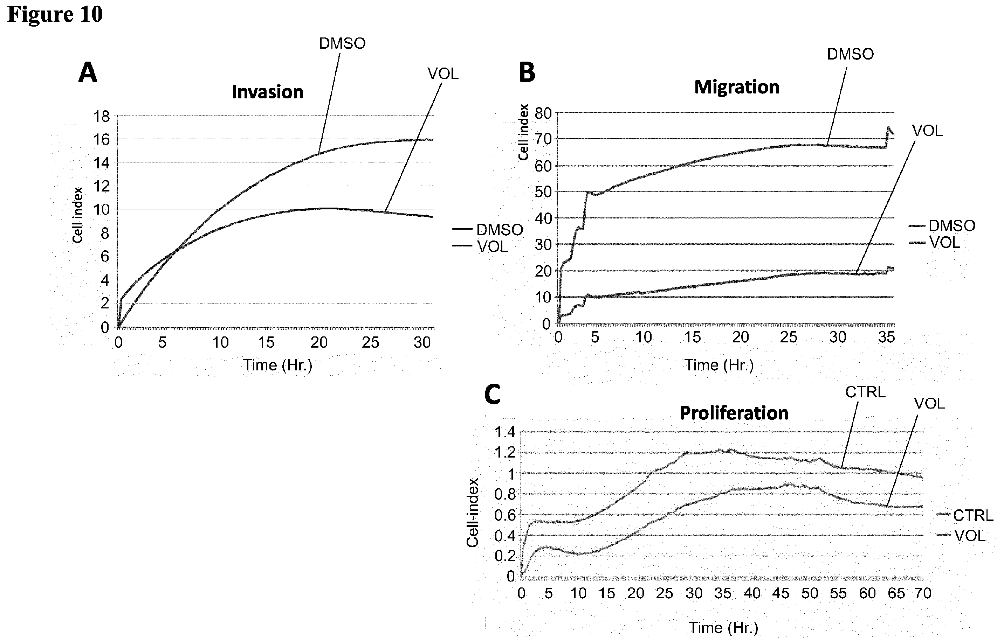

**Figure 10****:** The effect of volasertib on MDA-MB-231 behavior.
   (A)MDA-MB 231 cells were treated with DMSO and volasertib for 24 h. Then, cell invasion was monitored continuously over 30 hours using RTCA Software.
   (B) The same protocol was repeated without applying Matrigel to measure cellular migration.
   (C) MDA-MB-231 proliferation was monitored for 70 hours after treatment with 300 nM volasertib.

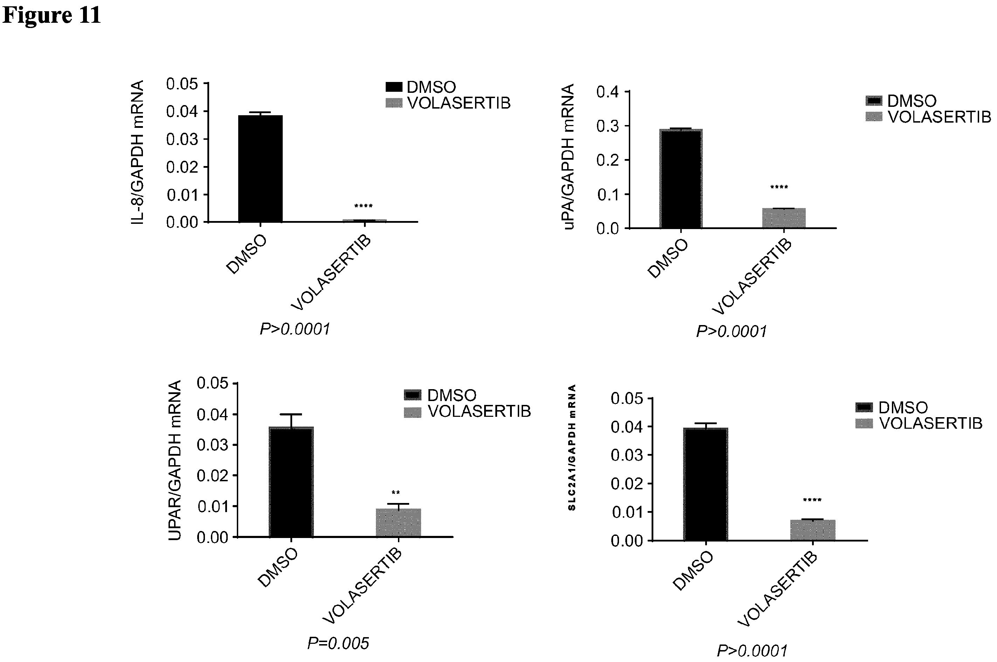

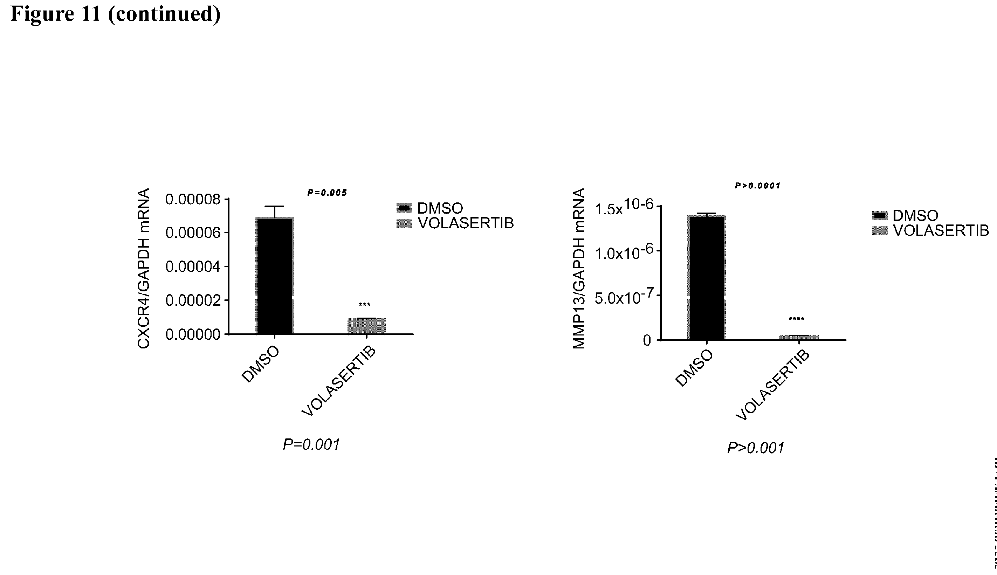

**Figure 11****:** Effect of volasertib on the expression of various cancer-related genes having ARE-containing mRNAs. mRNA expression of IL-8, uPA, uPAR, SLC2A1, CXCR4, MMP13 is shown for control cells and volasertib treated MDA-MB-231 cells. Values are shown as mean ± SEM and statistical analysis was performed using Student's *t*-test.

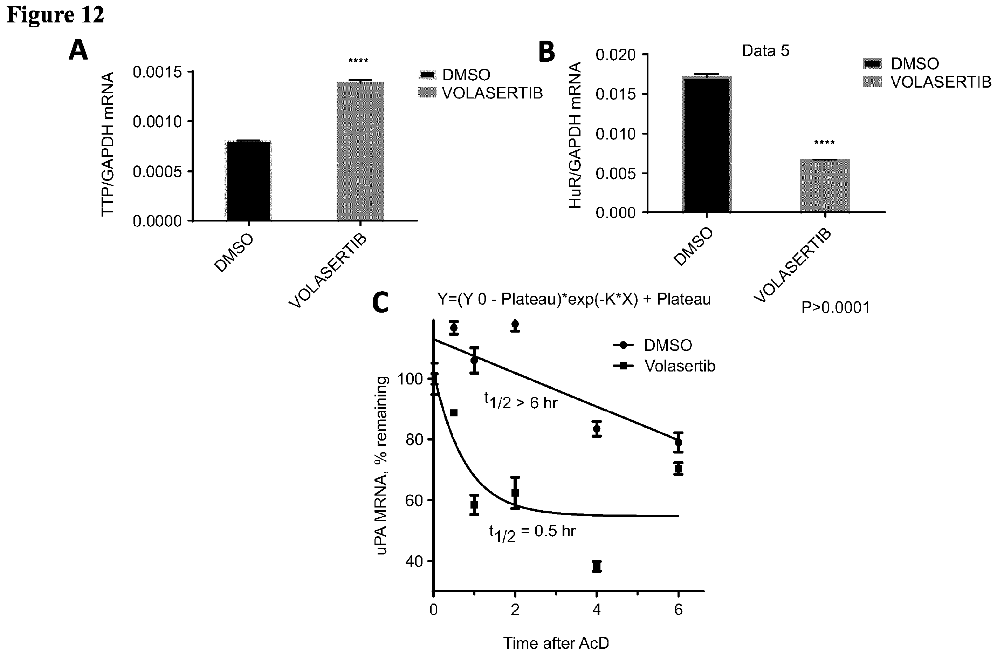

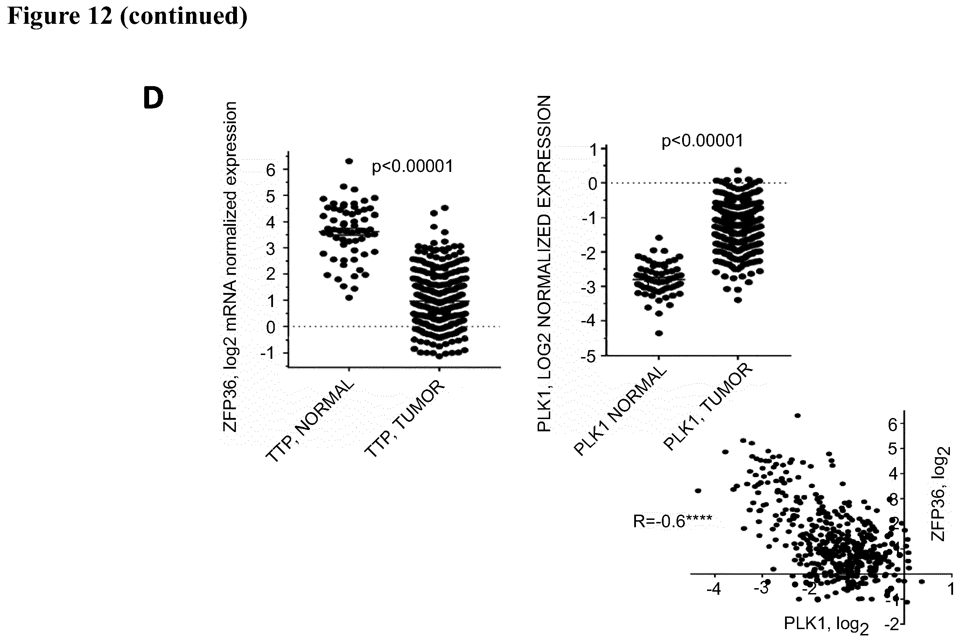

**Figure 12****:** Effect of volasertib on TTP expression and activity. MDA-MB 231 cells were treated with DMSO or volasertib for 24 h.
   (A) Effect of volasertib on *TTP* and
   (B) *HuR* mRNA expression; qPCR for *TTP* and *HuR* was performed: *****p*< 0.001.
   (C) mRNA decay curve for *uPA* in MDA-MB-231 cells using the one-phase exponential decay model.
   (D) The correlation between PLK-1 and TTP expression in cancer obtained from TCGA data, using the Oncomine portal. Values were expressed as mean ± SEM and comparison was performed using Student's t-test.

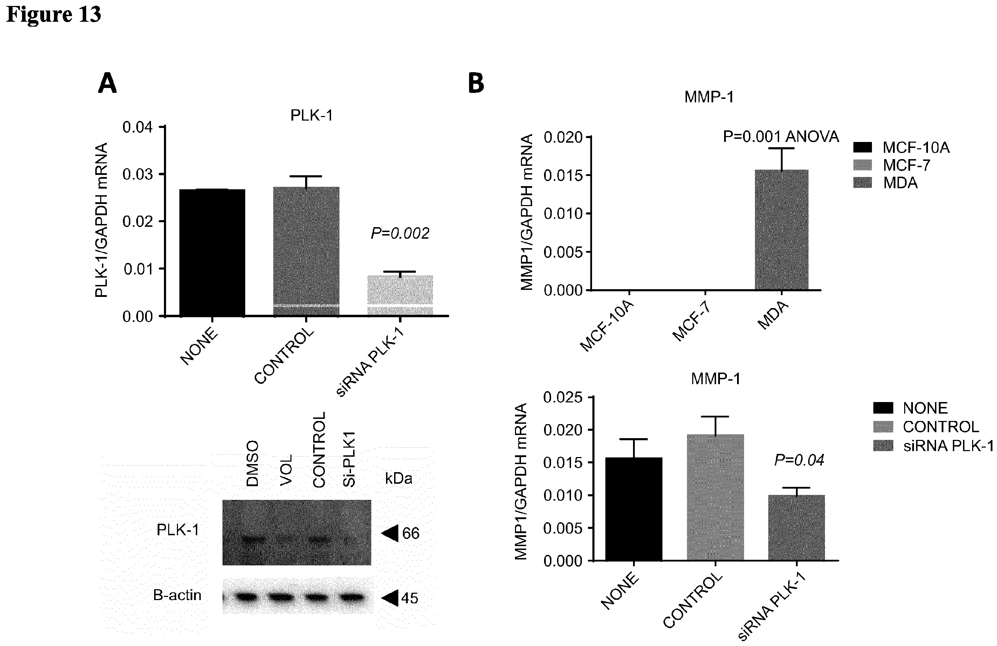

**Figure 13****:** Knockdown PLK-1 using siRNA in MDA-MB-231.
   (A) Expression of PLK-1 mRNA (upper panel) and protein (lower panel) after siRNA treatment.
   (B) Expression of MMP1 in normal cells (MCF-10A, non-tumorigenic cell line) and cancer cells (MCF-7, hormone responsive breast cancer cell line; and MDA, triple-negative breast cancer cell line) as shown in the upper panel, and after gene silencing of PLK-1 using siRNA (lower panel). Values are shown as mean ± SEM, and comparison was performed using Student's t-test.

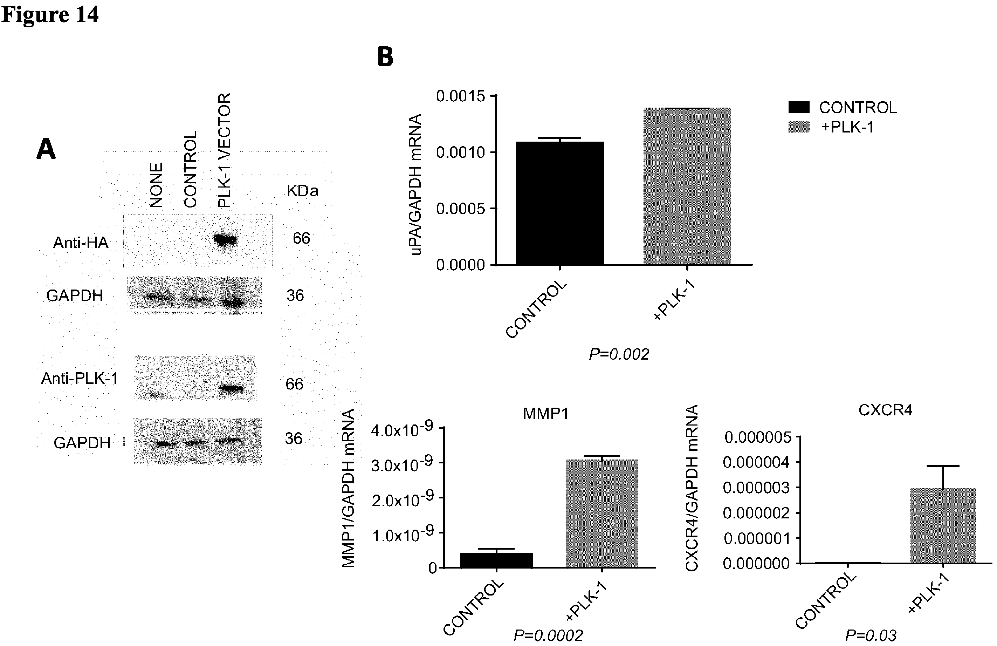

**Figure 14****:** PLK-1 overexpression in MCF10A Cells.
   (A) Expression of PLK-1 protein after transfection with PLK-1-vector.
   (B) mRNA expression of uPA, MMP1 and CXCR4. Values are shown as mean ± SEM, and comparison was performed using Student's t-test. +PLK-1 refers to PLK-1 overexpression; CXCR4 to chemokine receptor-4 (CXCR4); MMP1 to matrix metalloproteinase 1, and uPA to urokinase plasminogen activator.

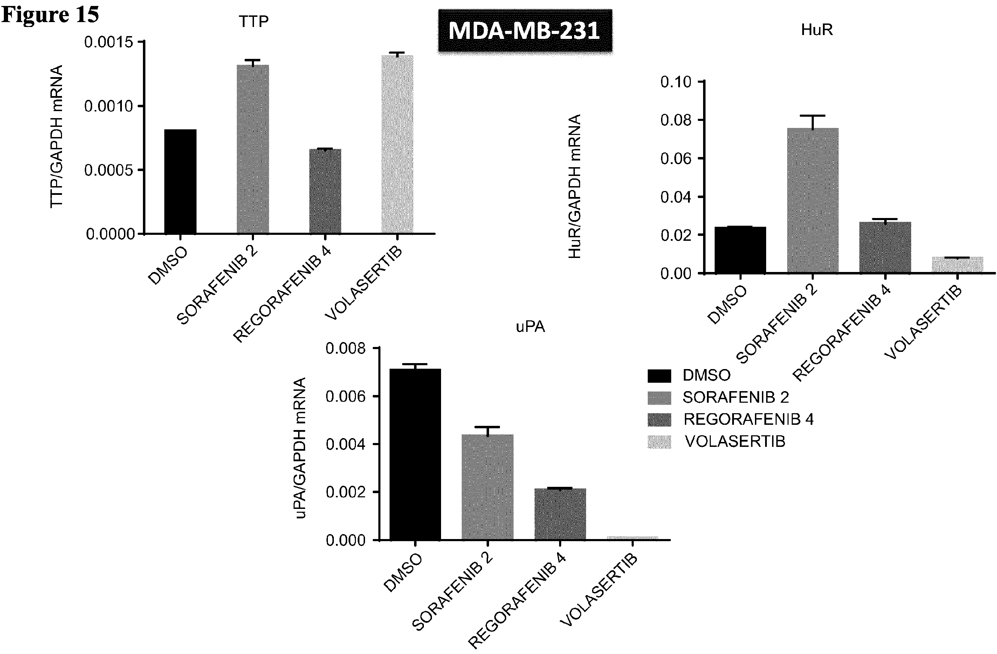

**Figure 15****:** Effect of protein kinase inhibitors sorafenib2, regorafenib4, and volasertib on expression of TTP, HuR, and uPA in MDA-MB-231 cells.

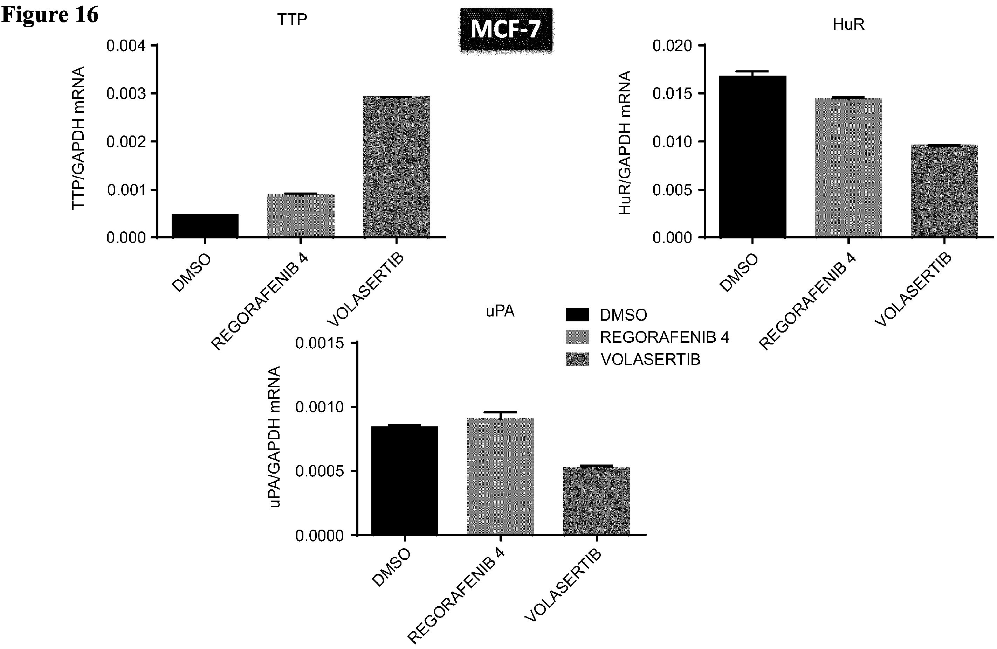

**Figure 16****:** Effect of protein kinase inhibitors regorafenib4 and volasertib on expression of TTP, HuR, and uPA in MCF-7 cells.

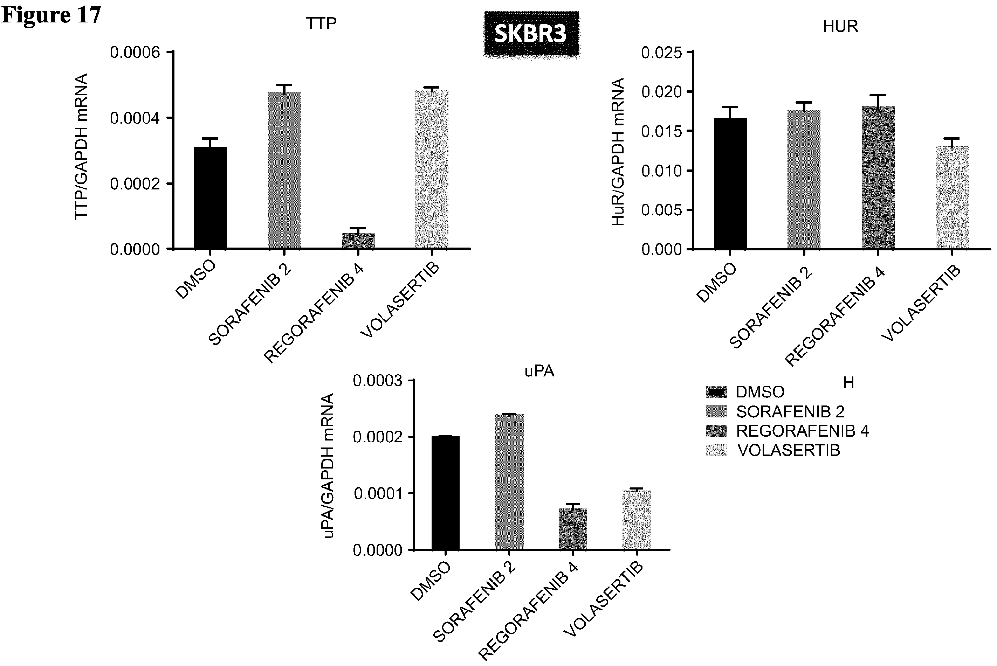

**Figure 17****:** Effect of protein kinase inhibitors sorafenib2, regorafenib4, and volasertib on expression of TTP, HuR, and uPA in SKBR3 cells.

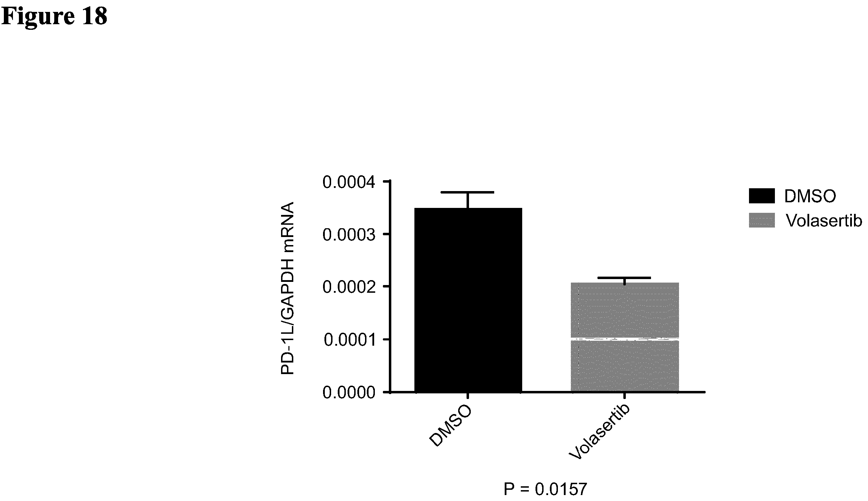

**Figure 18****:** Volasertib reduces PD-1L expression.

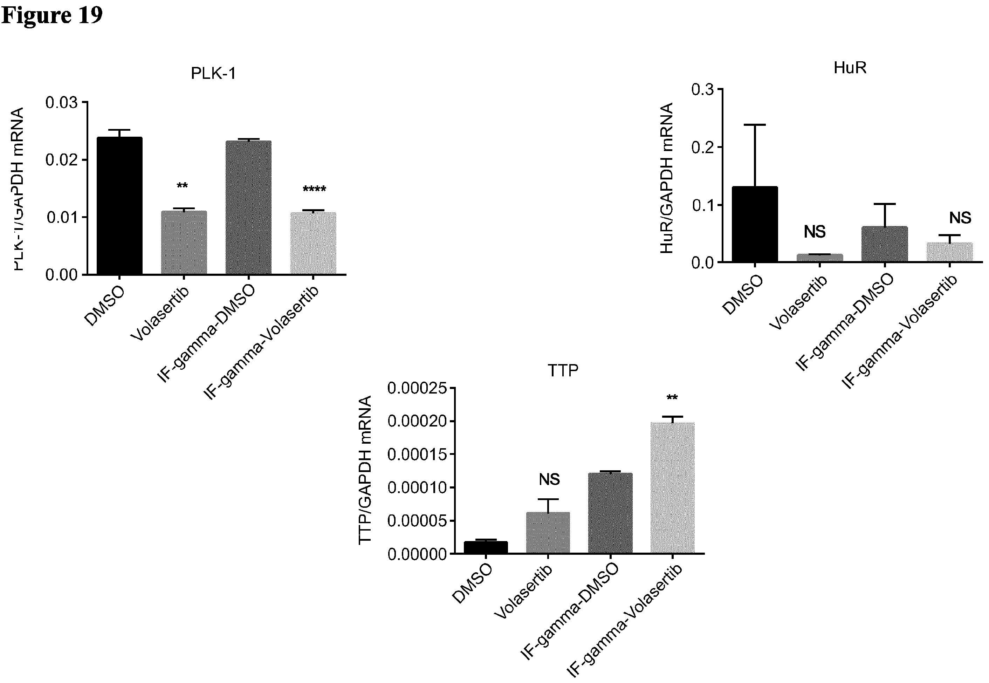

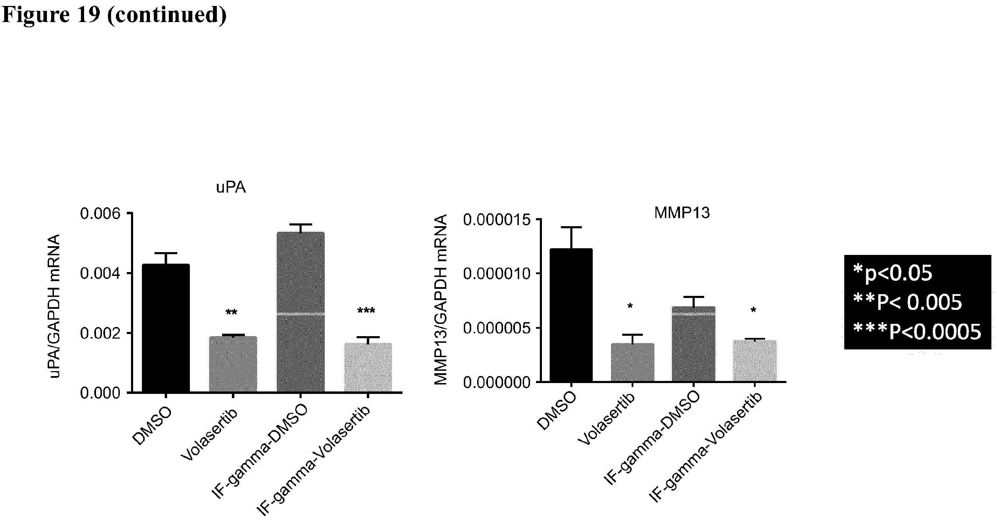

**Figure 19****:** Effect of volasertib and IFNγ on expression of PLK-1, TTP, HuR, uPA, and MMP13.

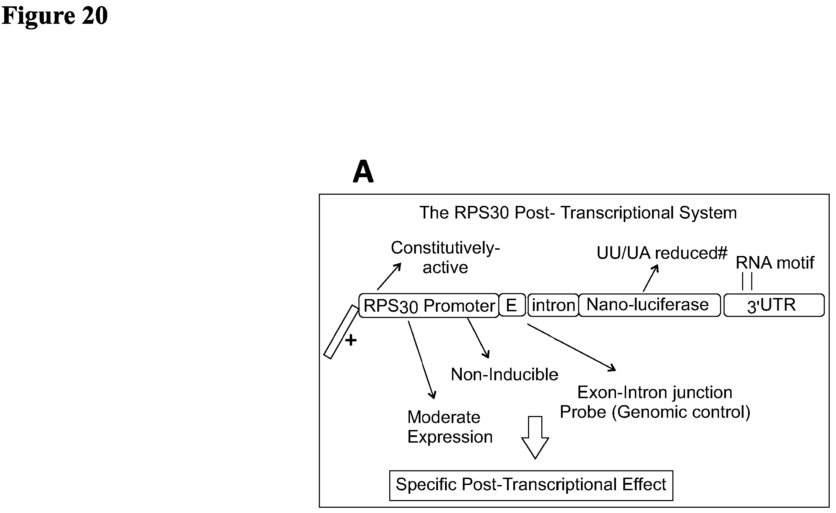

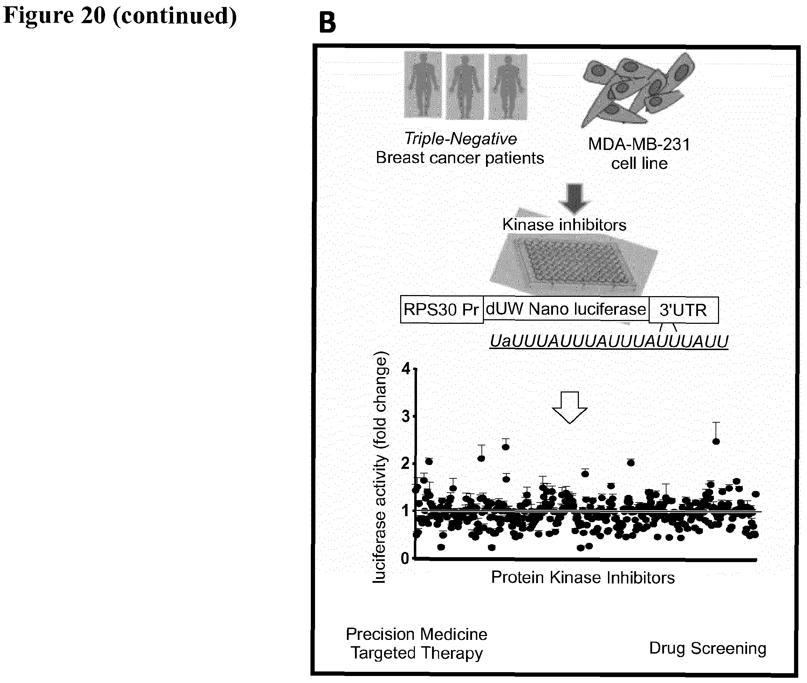

**Figure 20****:** Post-transcriptional control as a universal platform for kinase inhibitor drug screening.
   (A) Schematic representation of the highly sensitive and selective reporter vector system for the study of post-transcriptional gene expression. The system employs a constitutively active modified ribosomal protein promoter 30 (RPS30M1) and a reporter coding region, wherein said reporter coding region is preferably present in a modified form that has been modified by reducing UU/UA coding dinucleotides. The system allows for sensitive and selective assessment of 3' untranslated region (3'UTR)-mediated activity. The 3UTR contains AU-rich elements.
   (B) Schematic representation of an assay approach of the present invention using the reporter vector system with high-throughput capability which can be used to screen small molecule compounds, particularly kinase inhibitors for identifying drugs that target chronic inflammatory diseases and cancer, such as triple-negative breast cancer. MDA-MB-231 is a cellular model of triple-negative breast cancer. dUW represents a reduction of UU and UA dinucleotides in the coding regions.

### EXAMPLES

### Example 1: Kinome inhibitors screening on post-transcriptional gene regulation.

### Cell lines

Breast cancer cell lines MDA-MB-231, SKBR3, and MCF-7, and the normal-like breast cell line MCF10A were obtained from American Type Culture Collection (ATCC, Rockville, MD, USA). MDA-MB-231 and MCF-7 cell lines were cultured in Dulbecco's modified Eagle's medium (DMEM; Invitrogen, Carlsbad, CA, USA) at 37 °C supplemented with 2 mM glutamine and 10 % fetal bovine serum (FBS). SKBR3 cells were grown in McCoy's 5a Medium (McCoy's 5A; Thermo Fisher Scientific, Waltham, MA, USA) with 10 % FBS. MCF10A were maintained in Ham's F12-DMEM mixture (DMEM/F12 Ham; Thermo Fisher Scientific, Waltham, MA, USA) and supplemented with 20 ng/ml epidermal growth factor (EGF), 0.01 mg/ml bovine insulin and 500 ng/ml hydrocortisone (Sigma, St. Louis, MO, USA). All culture media were supplemented with 1 % penicillin-streptomycin antibiotics (Sigma, St. Louis, MO, USA). All transfections were performed in reduced serum media using Lipofectamine 2000 (Invitrogen).

### Reporter plasmids

Reporters were designed to be driven by the ribosomal protein subunit 30 (RPS30) promoter. The promoter was amplified using PCR with primers specific to the flanking region of the *RPS30* promoter sequence. The gene structure of RPS30 was obtained from the Ribosomal Protein Database (http://ribosome.med.miyazaki-u.ac.jp). The forward and reverse primers included the restriction sites, *EcoRV* and *SalI* sites. Amplified DNA fragments were then resolved in 1.2 %-1.5 % agarose gel and the amplicon bands were excised, purified and cloned into the promoterless plasmid. TNF-α 3'-UTR and TNF-α ARE were used to study the role of AU-rich elements in the action of PKs inhibitors. The AU-rich sequence that comprised 250 bases from TNF-α 3'UTR (1200-1450 bp, NM_00059) was amplified by PCR using the forward primer 5'-CAGCAGGATCCAGAATGCTGCAGGACTTGAG-3' (SEQ ID NO:1) and the reverse primer 5'-CGACCTCTAGACTATTGTTCAGCTCCGTTT-3' (SEQ ID NO:2). The TNF-α-ARE sequence was made by annealing two synthetic complementary oligonucleotides of 70 bases that correspond to TNF-α ARE. The control reporter was constructed to have the basic structure of post-transcriptional reporter except 3'UTR which lack the AU-rich element sequence like this of bovine growth hormone. Any ARE reiterations or variations can be used, with minimal sequence is AUUUA, preferably, WW(AUUUA)WW, where W=A or U. They can be also repeated as an overall, e.g., AUUUAUUA, anywhere from 0 to 10 repeats as example.

### Protein kinase inhibitors library

Selleckchem Kinase Inhibitor Library that includes a selection of 378 pharmacologically active inhibitors of a number of protein kinases was purchased from Selleckchem. (Houston, TX, USA). This library is a collection of 378 kinase inhibitors some of which have been approved by the FDA. Each compound is provided as a pre-dissolved 10 mM dimethyl sulfoxide (DMSO) solution as a 96 well format tube (100 µL). The compounds were diluted by OPTI-MEM (Thermo Fisher Scientific, Waltham, MA, USA) to a final concentration of 5 µM.

### Reporter assay

Post-transcriptional and control reporter constructs were transfected to MDA-MB-231 cells separately that were seeded in 96-well microplates at a density of 4 × 10⁴ cells/well and incubated overnight. Using lipofectamine 2000 protocol (Invitrogen, Carlsbad, CA, USA), the cells were transfected with 10 ng of either RPS30-luciferase-control 3'UTR or RPS30-luciferase-ARE 3'UTR reporter plasmids. After 24 h, the cells were treated with each member of protein kinase inhibitors kit for 24 h. Then, luciferase reaction was performed as prescribed by manufacture's protocol (Nano-Glo Luciferase, Promega, Madison, WI, USA)/well. After 15 minutes, the reporters' activities were measured through the measurement of the chemiluminescence (Figure 8) after treatment using a Zenith 3100 (Anthos Labtec, Eugendorf, Austria).

### Statistical analysis

Data are presented as means ± standard error of the mean (SEM). Two-sample Student's t-test was used to determine the differences between two data sets. One-way analysis of variance (ANOVA) was used to compare three or more data columns. Two-way analysis of variance was used to analyze two groups of data, each having two data columns. Analysis was performed using GraphPad Prism version 6.00 for Windows, (GraphPad Software, La Jolla California, USA). For high throughput screening (HTS) hit selection (protein kinase inhibitors), strictly standardized mean difference (SSMD) test was used. This statistical test measures the size of effect of each member in a group relative to the other members. It is the mean of each member divided by the standard deviation of the difference between two random values from different groups. Based on the value of SSMD, the effect of each member of the library was classified as strong (*β* ≥ 5), moderate (1≤ *β* <5), and weak (*β* < 1). In this study, only drug with SSMD score more than or equal to 5 or less than or equal to -5, i.e. strong *β*, where selected in the primary screening.

### PLK-1 regulates ARE-mediated post-transcriptional pathways in breast cancer

MDA-MB-231 cells are a model for highly invasive breast carcinomas and characterized by aberration in several protein kinase pathways including ERK, PI3K, MAP kinases, Ras, and many receptor and nonreceptor tyrosine kinases. To perform a functional kinome screen, the PK inhibitors were examined for their effect on the expression of ARE and non-ARE containing reporter using optimized and highly selective post-transcriptional reporter system as set forth above. The screening was performed in three stages to detect the protein kinase inhibitors that potentially affect the phosphorylation of an ARE-BP, as shown by lowering of the expression of ARE-containing reporter activity. In the primary screening, 87 out of 378 drugs in the PK library were found to reduce the expression of ARE-containing reporter without comparing their effect to a control reporter. Only three of the protein kinase inhibitors were confirmed to be involved in ARE-dependent mRNA regulation in the later stages of screening when they were compared to a non-ARE reporter. These potent protein kinase inhibitors were AZ628, Regorafenib, and Volasertib, and their substrates are Raf, VEGFR2 and PLK-1 respectively (Figure 8B).

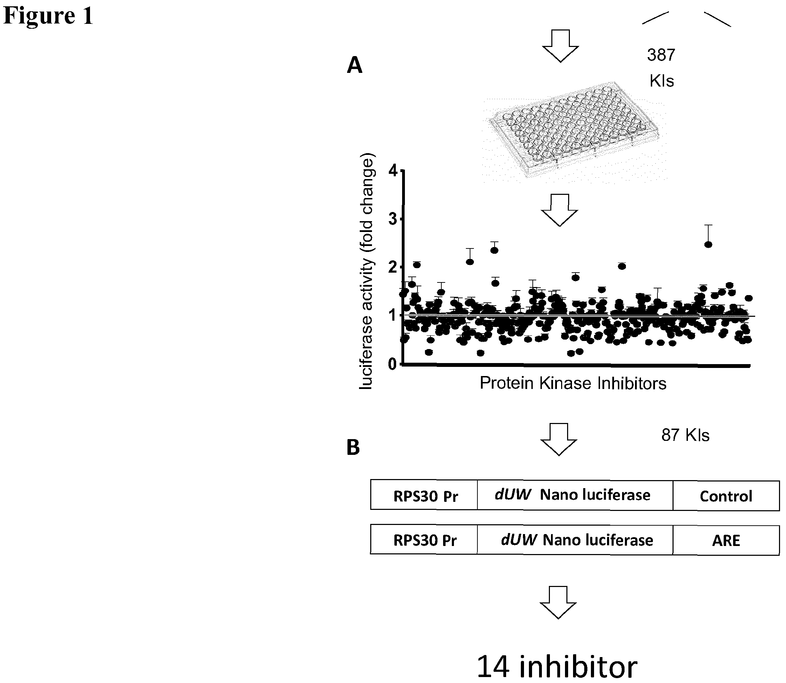

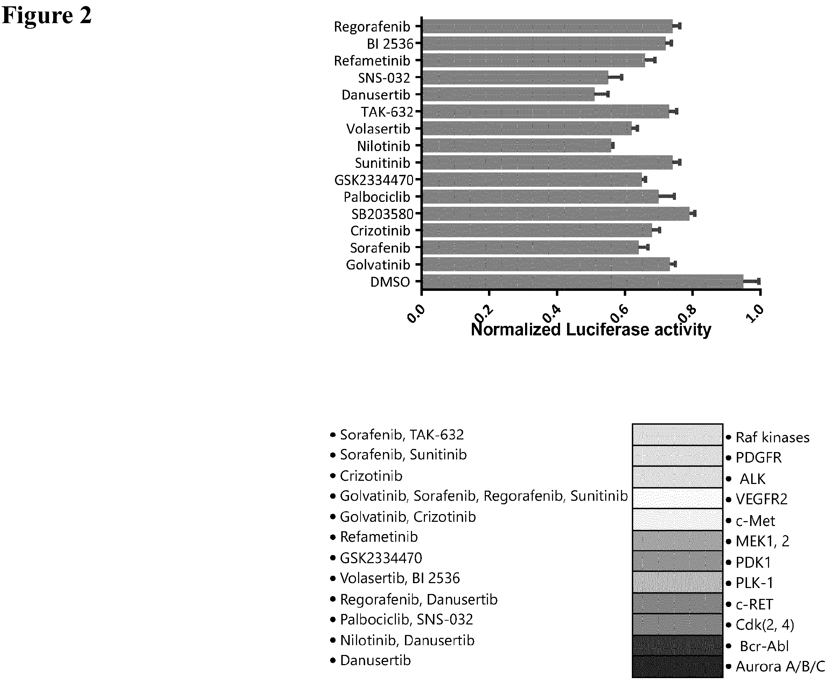

First, a "primary screening" was performed in which MDA-MB-231 cells were transfected with the post-transcriptional luciferase reporter with 3'UTR-containing ARE and then treated with 5 µM/well of each member in the PK inhibitor library or DMSO as a vehicle control for 16 hr (Figure 1A). Finally, the drugs that specifically reduced the expression of the ARE reporter in comparison to the non-ARE reporter were selected in a second stage which aimed at narrowing the primary screen results to eliminate non-specific effects on non-ARE reporter activity, and these were subjected to further investigation. In this stage, the cells were transfected with both the ARE and non-ARE control post-transcriptional reporters and treated with different doses of the protein kinase inhibitors (0.5, 2, and 5 µM concentrations). Several drug groups were discovered that reduce ARE-post-transcriptional reporter activity. Fig. 2 shows a list of 15 protein kinase inhibitors that have activity against the specific cancer type/sub-type. Figure 2), including for examples, namely rapidly accelerated fibrosarcoma kinases (B-Raf), VEGF receptors type 2 (VEGFR2), and polo-like kinase 1 (PLK-1), comprising AZ 628 (pan-Raf inhibitor), Regorafenib (BAY 73-4506), inhibiting Raf-1 and VEGFR1-3, and Volasertib (BI 6727, PLK-1 inhibitor).

### Example 2: Expression of PLK-1 in normal cells and cancer cells.

Methods were performed as described in the foregoing example.

### Plasmids and RNA interference

Vector used for *PLK-1* overexpression was obtained from Genecopoeia (Rockville, Maryland, United States) and had been designed to have human influenza hemagglutinin (HA) tag. RNA interference studies were performed using chemically synthesized siRNA duplexes purchased from Santa Cruz (Santa Cruz Biotech, CA) for silencing of *PLK-1* and a control siRNA. Western blotting and RT-PCR were utilized to determine the efficiency of siRNA silencing after 48 hr. Lipofectamine LTX was used for siRNAs transfection following the manufacturer protocol (Invitrogen, Carlsbad, CA, USA). The final concentration of siRNAs used for transfection was 50 nM.

### PLK-1 is overexpressed in cancer cells

The expression of PLK-1 in normal MCF-10A and two breast cancer cell lines, MDA-MB-231 and MCF-7, was measured using RT-PCR. As shown in Figure 9, breast cancer cells exhibited moderate to high expression of PLK-1 compared to normal cell line. The MDA-MB-231 cells significantly express higher levels of PLK-1 mRNA than normal breast epithelial cells. Concurrently, the triple negative breast cancer subtype is characterized by higher expression of PLK-1 compared to the other types of breast cancer according to data obtained from TCGA data (Figure 9D), using the Oncomine portal. Patients' data also revealed that PLK-1 is overexpressed in a wide range of human cancers including breast, liver, lung, prostate, kidney, gastric and bladder carcinomas (Figure 9C). High expression of PLK-1 was found to be associated with reduced survival among cancer patients. (Figure 9E).

### Example 3: Protein kinase inhibitors reduces cancer and inflammatory cytokine mRNA expression.

Methods were performed as described in the foregoing examples.

Examples of the effect of several protein kinase inhibitors that were the outcome of the screen assay on one cancer gene, uPA, and a pro-inflammatory cytokine, IL-8 is given in Fig. 3. All seven-member protein kinase inhibitor group significantly reduced uPA mRNA abundance in MDA-MB-231 cell line (Fig. 1D). The majority of this group reduces the mRNA abundance of IL-8 (except sorafenib), The MDA-MB-231 cells were treated with 330 nM of each of the inhibitor or DMSO as control for 24 hr, then quantitative RT-qPCR was performed in using FAM-labelled TaqMan probe of the above genes and normalized to GADPH (Figure 3).

Furthermore, as an example, the effect of the PLK1 inhibitor volasertib was studied on the expression of known genes that have been shown to be upregulated in cancer and bearing ARE sequence in their mRNA. These include IL-8, solute carrier family 2 member 1 (SLC2A1), uPA, uPAR, CXCR4, MMP13, PDL1, and HuR. The MDA-MB-231 cells were treated with 330 nM volasertib or DMSO for 24 hr, then quantitative RT-qPCR was performed in using FAM-labelled TaqMan probe of the above genes and normalized to GADPH (Figure 11). As can be observed from Figure 11, the expression of all ARE-containing cancer genes was significantly reduced using volasertib.

### mRNA half-life and quantitative reverse transcription polymerase chain reaction

Cells were cultured in six-well plates and either treated with DMSO or drugs for 24 h. Total RNA was then extracted using Trizol reagent (TRI Reagent, Sigma-Aldrich, St Louis, MO). The cells were lysed directly on the culture dish by adding 1 ml of the TRI Reagent per 10 cm² surface area. Reverse transcription for preparation of cDNA was performed using 3 µg of total RNA, 150 ng random primers, 0.1 M dithiothreitol (DTT), 10 mM deoxynucleotide triphosphate (dNTP) and 200 U of SuperScript II (Invitrogen, Foster City, CA). The quantitative RT-QPCR was performed in multiplex in the Chroma 4 DNA Engine cycler (BioRad, Hercules, CA, USA) using FAM-labelled TaqMan probes (Applied Biosystems, Foster City, CA, USA) for TTP (*ZFP36*)*,* HuR (*ELAVL1),* uPA (*PLAU*)*,* ,CXCR4, MMP-1, MMP-13, IL-8, PLK-1 while a VIC-labelled glyceraldehyde-3-phosphate dehydrogenase (*GAPDH)* probe was used as the endogenous control. Samples were amplified in triplicate and quantification of relative expression was performed using the estimation of quantitation cycle (Cq) method.

For half-life experiments, 5 µg/ml of Actinomycin D (ActD; Sigma-Aldrich, St Louis, MO) was added to the cells for 1, 2, 4 and 6 h prior to extraction of total RNA using Trizol. The reverse transcription reaction and quantitative PCR were performed as described above. The half-life of mRNAs was estimated using the one-phase exponential decay method using GraphPad Prism software (GraphPad Software, San Diego, CA).

### Western blotting

The cells were lysed in a mixture of 2 x Laemmli buffer (BioRad, Hercules, CA, USA) and DTT (1:1). The cell lysates were loaded and subjected to electrophoresis on 4-12 % NuPAGE Bis-Tris gel (Invitrogen, Foster City, CA, USA). Rainbow protein molecular weight marker was used as a ladder to detect the size of the proteins. Then, the proteins were transferred from the gel to nitrocellulose membranes (Hybond ECL; Amersham Biosciences, Piscataway, NJ) in the presence of NuGAGE 20 x transfer buffer (Invitrogen, Foster City, CA, USA). After blocking, membranes were incubated with primary antibodies diluted in 5 % bovine serum albumin (BSA) (Sigma-Aldrich, St Louis, MO) 4 °C overnight. Antibodies used include rabbit anti-PLK-1, rabbit anti-caspase 3, rabbit anti-Bcl2, rabbit anti-actin (dilution 1:1000, Cell signaling, Massachusetts, USA). Then after, the membranes were incubated with enzyme (e.g. horseradish peroxidase, HRP) conjugated with goat anti-rabbit or anti-mouse secondary antibodies (diluted in 5 % BSA, 1:2000 dilution) (Santa Cruz Biotech, Santa Cruz, CA) for 1-3 hr. Protein bands were detected using ECL Western blotting detection reagents (Amersham Biosciences, Amersham, UK) in Molecular Imager ChemiDoc machine (BioRad, Hercules, CA, USA).

### Example 4: Invasion, migration, and proliferation of cells

Methods were performed as described in the foregoing examples.

### Invasion and migration assays

MDA-MB-231 cells were seeded in 6-well cell culture plates and incubated overnight. The cells were treated with the selected protein kinase inhibitors and incubated overnight. Then they were reseeded onto the invasion chamber in serum-free media at a density of 2 x 10⁴ cells per well using 16-well CIM-Plate in Real-Time Cell Analysis (RTCA) Dual Plate (DP) Analyzer (ACEA Bioscinece, California, USA) that was loaded inside the incubator. The lower chambers to which cells will migrate were prepared to contain chemoattractant consisted of 10 % FBS. For invasion assays, Matrigel (BioCoat, BD Biosciences, MA) which resembles the complex extracellular environment found in many tissues was prepared and used to coat the upper chamber of CIM plates. Invasion and migration were monitored continuously over 72-hour period using RTCA Software (ACEA Bioscinece, California, USA). The RTCA Instrument automatically monitors the cells every 15 minutes for 100 repetitions. For proliferation assays, the same principle was applied using a single chamber containing complete DMEM media (Figure 10).

### Volasertib inhibits invasion, migration and proliferation of breast cancer cells

Based on the dose-response curve, 330 nM was selected to be used as a standard dose for the experiments. In the invasion assay, MDA-MB-231 cells were treated with volasertib or DMSO for 24 hr, then invasion was monitored for 30 hr. As shown in (Figure 10A), MDA-MB-231 cells showed a reduced invasive behavior after treatment with volasertib compared to the control DMSO and the effect started as early as 6 hr after treatment. Similarly, volasertib was shown to reduce breast cancer cells migration (Figure 10B) for thirty hours after treatment. Proliferation of MDA-MB-231 was found to be significantly reduced after treatment with volasertib (300 nM) compared to DMSO (Figure 10C).

PLK1 kinase, as an example of kinases involved in cancer, is over-expressed in breast cancer cell lines, including MCF7, MDA-MB-231 and SKBR3 when compared to the normal-like MCF10A (Fig. 5A). Many kinases are either over-expressed or constitutively active or hyperactive in cancer states when compared to normal cells. As example of inhibitors that are found in the precision cancer therapy screen, Volasertib and Regorafenib (VEGFR kinase inhibitor) led to reduction in the level of ARE-containing mRNA, uPA in most of the cancer cell lines. (Fig. 5B).

### Global effects of PLK1 inhibition on gene expression.

mRNA was extracted from DMSO- or volasertib-treated MDA-MB-213. cDNA libraries were made for sequencing a measure of transcript copy numbers that correspond to the abundance of each gene (Fig. 6A). There was a total of 540 genes in which their expression is down-regulated by at least 1.7-fold reduction (p<0.001)-Figure 6B. Among those is 170 ARE-coding genes as derived by crossing with AU-rich element database. (Fig. 6B). Functional enrichment analysis show that the most affected groups belong to cell cycles and cytokines (Fig. 6C). Examples of these cytokines that were down-regulated is IL-8, uPA, IL-6, IL-11, IL1β, CSF2, and endothelin-1 and 2. Examples of cell cycle (cellular growth) regulators that are down-regulated: CCNE2, CDC6, E2F1, and MCM10.

### Example 5: Correlation of PLK1 expression and AU-rich mRNA expression.

Methods were performed as described in the foregoing examples.

### Patient data and analysis

The Cancer Genome Atlas (TCGA) was searched using the Oncomine web portal, www.oncomine.com. TCGA is collaboration between the National Cancer Institute (NCI) and National Human Genome Research Institute (NHGRI) that provides maps for the genomic changes in different types of cancer. According TCGA, they have declared the following: 'All samples in TCGA have been collected and utilized following strict human subjects protection guidelines, informed consent'. As an example, the present inventor shows that TCGA dataset of invasive ductal breast cancer was used. The expression levels of PLK1 from nearly 300 patients' data were obtained and the expression of those genes that are conform to the following criteria were obtained: a) ARE-mRNA, (b) over-expressed in cancer by at least 1.7-fold (p<0.001), and (c) down-regulated by PLK1 inhibitor (data from Fig. 6). There were 40 genes as such and include the following: (INHBA, MCM10, CDC6, DTL, ZNF367, E2F1, E2F8, CDC25A, RAD54L, MCM6, CHML, MYBL1, PLAUR, OAS2, RTKN2, PRSS22, MTBP, WDR76, DNA2, LRRCC1, LSM11, PLAU, ELAVL1, EDN2, BMPR1B, IGSF8, MAPK13, SFXN2, BARD1, MEX3A, PLEKHA6, TMEM184A, JPH1, SLC16A6, PRRG4, POLD3, IL11, IL8, and CCNE2). The average of all the expression levels of these genes were plotted against PLK1 expression levels from each of the patient data (Fig. 7). There was a tight correlation co-efficient (r=0.8; p<0.0001, Spearman's correlation test).

### Volasertib increases TTP expression and reduces HuR

PLK-1 inhibition using volasertib in MDA-MB-231 cells resulted in 40% enhancement of *TTP* and 60% reduction of *HuR* mRNA expressions (Figure 12A,B). To study the influence of PLK-1 inhibition on ARE-containing mRNA stability, the effect of volasertib treatment on the half-life of uPA was analyzed. Half-life of uPA mRNA was reduced from > 6 hr to 0.5 hr in response to volasertib treatment compared to the DMSO control (Figure 12C). The relationship between PLK-1 and TTP expression in normal and cancer patients was analyzed and as shown in Figure 12D, patients having a TNBC tumor have high PLK-1 and low TTP levels. Compatible with the findings of the inventors, the higher the level of PLK-1, the lower the TTP expression.

### PLK-1 silencing reduces the expression of TTP targets

To investigate whether the effects produced by volasertib are attributed to PLK-1 inhibition specifically, siRNA was used to knock-down PLK-1 in MDA-MB-231 cells. After transfection, the gene expression and protein level of PLK-1 were monitored to ensure the efficiency of PLK-1 siRNA (Figure 13A). The influence of PLK-1 silencing on ARE-BPs action was verified by measuring one of three ARE-bearing targets, namely MMP-1. Expression of MMP-1 was found to be significantly high in MDA-MB-231 cell line and nearly undetectable in normal MCF-10A and cancerous MCF-7 cells (Figure 13B, upper panel). Treatment with PLK-1 siRNA significantly lowered the level of MMP-1 mRNA compared to the control siRNA in MDA-MB-231 cells (Figure 13B, lower panel).

### PLK-1 overexpression inhibits the action of TTP

For further characterization of the role of PLK-1 in ARE-mediated regulation of post-transcription, we overexpressed PLK-1 in normal MCF10A breast cells and measured the expression of some ARE-containing genes. Overexpression of PLK-1 in MCF10A cells was verified by western blotting (Figure 14A). As shown in Figure 14B, the overexpression of PLK-1 in MCF10A cell was associated with moderate increase in uPA mRNA expression. As expected, the expression of MMP1 and CXCR4 was very low in normal cells, yet PLK-1 overexpression increased their levels.

The present inventor discloses a method of treatment of cancer using ARE-mediated gene post-transcriptional regulation involving a protein kinase that is aberrant in cancer. Examples were given in terms of PLK1 protein as it was shown by Western blotting that it is over-expression (Fig. 12A). There are many protein kinases that are over-expressed in cancer when compared to normal cells. These can at mRNA level, protein abundance level, or activity level. PLK-1 inhibitors volasertib and BI 2536 reduced the TNF-ARE luciferase reporter activity by 40 % and 35 %, respectively. With regard to volasertib, the same reduction in percent reporter activity was observed in the secondary screening compared to the control reporter, wherein with regard to BI 2536, the reduction in percent reporter activity was reduced to 16 % in the secondary screening. Other PLK inhibitors including rigosertib, HMN-214, GSK461364, Ro3280 and NMS-P937 did not reduce ARE-reporter activity. Rigosertib is a PLK-1 inhibitor but shows more than 30-fold selectivity against PLK-2 with no activity on PLK-3 whereas GSK461364 and NMS-P937 are PLK-1 inhibitors in phase 1 trial with more than 1000-fold activity against PLK2 and PLK-3.

The present inventors disclose that triple negative breast cancer cells express significantly higher levels of PLK-1 mRNA and protein compared to HER negative cancer and normal breast epithelial cells. The present inventors further disclose that the effect of PLK-1 inhibition is consistent among different types of breast cancer including triple-negative, HER negative, and ER-PR negative breast cancers, as shown through the actions induced by PLK-1 inhibitor volasertib on MDA-MB-231, MCF7, and SKBR3 cell lines, namely increasing the expression of TTP, decreasing the HuR level, and downregulating the TTP target uPA. The present inventors disclose that triple-negative breast cancer cell proliferation is significantly reduced by volasertib. The present inventor further discloses that invasion and migration of MDA-MB-231 were significantly reduced and cancer-related genes, such as CXCR4, MMP1, MMP13, uPA, and uPAR, were downregulated upon PLK-1 inhibition. Accordingly, the present inventor discloses that any protein kinase inhibitor which post-transcriptionally regulates expression of cancer-related genes is suitable for precision cancer therapy. Further disclosed is a method of treatment of cancer comprising administering said protein kinase inhibitor, to a patient in need thereof.

The present inventors further disclose that PLK-1 inhibition reduces the half-life of TTP target uPA, and that PLK-1 inhibitor volasertib reduces expression of ARE-containing mRNA targets, such as CXCR4, IL-8, MMP1, MMP13, uPA, uPAR, and SLC2A1 mRNA. In addition, the present invention discloses that volasertib increases the TTP level and reduces the HuR level. Correspondingly, PLK-1 overexpression resulted in increasing the expression of TTP targets, such as uPA, MMP1, and CXCR4.

The present invention discloses the effect of a wide range of protein kinases on ARE-mediated regulation of gene expression, in particular three kinase pathways are disclosed to be involved in ARE-dependent mRNA regulation, including Raf, VEGFR2 and PLK-1. PLK-1 is disclosed to regulate the expression of many ARE-containing mRNAs especially those involved in cancer by phosphorylation of ARE-BPs. PLK-1 inhibitor volasertib is disclosed to reduce the level and activity of an ARE-containing reporter and to reduce the half-life of ARE-containing uPA mRNA. PLK-1 inhibition is disclosed to normalize TTP deficiency and HuR overexpression in breast cancer, and inhibited invasive breast cancer cell proliferation, migration and invasion. The present invention discloses a method of treatment of cancer comprising PLK-1 inhibition, wherein PLK-1 inhibition normalizes the TTP/ HuR ratio and inhibits cancer cell proliferation, migration and invasion. These events are involved in hallmarks of cancer including cell division and growth, angiogenesis, glycolysis, apoptosis resistance, invasion, and metastasis. In addition, the benefit of controlling inflammatory cytokine release as a result of the use of the specific kinase inhibitor since this "Precision oncology assay", which is the method of identifying a protein kinase inhibitor for normalizing post-transcriptional regulation as precision cancer therapy, detects reduction in gene expression related to uncontrolled cell cycle, other cancer processes, and additionally inflammatory cytokine release culminating in wider benefit to the patients.

### Example 6: Post-transcriptional control as a universal platform for kinase inhibitor drug screening.

Methods were performed as described in the foregoing examples.

Abnormal post-transcriptional control of gene expression contributes to sustained and excessive production of pro-inflammatory and cancer-promoting cytokines, growth factors, and other mediators. The present inventors have developed and optimized a highly sensitive and selective reporter vector system for the study of post-transcriptional gene expression (Figure 20). The present inventor used this optimized system to find small molecule drugs that target AU-rich element (ARE)-mediated pathways. AREs are key determinants of mRNA stability and translation, and aberrations in ARE-mediated pathways occur during carcinogenesis and inflammatory diseases. 1000+ FDA-approved drugs were screened for their effect on ARE-reporter expression in cancer cells. The present inventor found that merely glucocorticoids were capable of such activity indicating high selectivity. Furthermore, the present inventors tested a protein kinase inhibitor library comprising about 400 protein kinase inhibitors in a cellular model of triple-negative breast cancer using MDA-MB-231 cells. The present inventors were able to identify several groups of protein kinase inhibitors showing an effect in MDA-MB-231 cells. These results pave the way for targeting a cancer, such as triple negative breast cancer, with high precision.

### Example 7: Exemplary protein kinase inhibitors.

The list in Table 1 below shows examples of protein kinase inhibitors which can be tested in a method of identifying a protein kinase inhibitor for normalizing post-transcriptional regulation as precision cancer therapy.

**TABLE 1. EXAMPLES OF KINASE INHIBITORS AND THEIR TARGETS.**

| Kinase inhibitor | Target |
|---|---|
| (-)-BAY-1251152 | CDK |
| (-)-Indolactam V | PKC |
| (+)-BAY-1251152 | CDK |
| (±)-Zanubrutinib | Btk |
| (1S,3R,5R)-PIM447 (dihydrochloride) | Pim |
| (3S,4S)-Tofacitinib | JAK |
| (E)-AG 99 | EGFR |
| (E)-Necrosulfonamide | Mixed Lineage Kinase |
| [6]-Gingerol | AMPK; Apoptosis |
| 1,2,3,4,5,6-Hexabromocyclohexane | JAK |
| 1,3-Dicaffeoylquinic acid | Akt; PI3K |
| 1-Azakenpaullone | GSK-3 |
| 1-Naphthyl PP1 | Src |
| 1-NM-PP1 | PKD |
| 2,5-Dihydroxybenzoic acid | Endogenous Metabolite; FGFR |
| 2-D08 | c-RET,SUMO,TAM Receptor,IL Receptor,PI3K,VEGFR,GSK-3 |
| 2-Deoxy-D-glucose | Hexokinase |
| 2-Methoxy-1,4-naphthoquinone | PKC |
| 2-Phospho-L-ascorbic acid trisodium salt | c-Met/HGFR |
| 3,4-Dimethoxycinnamic acid | ROS |
| 3BDO | Autophagy; mTOR |
| 3-Bromopyruvic acid | Hexokinase |
| 3-Methyladenine (3-MA) | Autophagy,PI3K |
| 4µ8C | IRE1 |
| 5-Aminosalicylic Acid | NF-κB; PAK; PPAR |
| 5-Bromoindole | GSK-3 |
| 5-Iodotubercidin | Adenosine Kinase |
| 6-(Dimethylamino)purine | Serine/threonin kina |
| 6-Bromo-2-hydroxy-3- | |
| methoxybenzaldehyde | IRE1 |
| 7,8-Dihydroxyflavone | Trk Receptor |
| 7-Hydroxy-4-chromone | Src |
| 7-Methoxyisoflavone | AMPK |
| 8-Bromo-cAMP sodium salt | PKA |
| A 419259 (trihydrochloride) | Src |
| A 77-01 | TGF-β Receptor |
| A 83-01 sodium salt | TGF-β Receptor |
| A-443654 | Akt |
| A-484954 | CaMK |
| A66 | PI3K |
| A-674563 | Akt,CDK,PKA |
| A-769662 | AMPK |
| ABBV-744 | Epigenetic Reader Do |
| Abemaciclib | CDK |
| Abrocitinib | JAK |
| ABT-702 dihydrochloride | Adenosine Kinase |
| AC480 (BMS-599626) | EGFR,HER2 |
| AC710 | c-Kit; FLT3; PDGFR |
| Acalabrutinib (ACP-196) | BTK |
| Acalisib | PI3K |
| acalisib (GS-9820) | PI3K |
| ACHP (Hydrochloride) | IKK |
| ACTB-1003 | FGFR; VEGFR |
| Acumapimod | p38 MAPK |
| AD80 | c-RET,Src,S6 Kinase |
| Adavosertib | Wee1 |
| AEE788 | EGFR |
| Afatinib | Autophagy; EGFR |
| Afatinib (BIBW2992) | EGFR,HER2 |
| Afatinib (dimaleate) | Autophagy; EGFR |
| Afuresertib | Akt |
| AG 555 | EGFR |
| AG-1024 | IGF-1R |
| AG126 | ERK |
| AG-1478 | EGFR |
| AG-18 | EGFR |
| AG-490 | Autophagy; EGFR; STAT |
| Agerafenib | Raf |
| AGL-2263 | Insulin Receptor |
| AICAR | AMPK; Autophagy; Mitophagy |
| AIM-100 | Ack1 |
| AKT inhibitor VIII | Akt |
| AKT Kinase Inhibitor | Akt |
| Akt1 and Akt2-IN-1 | Akt |
| Akti-1/2 | Akt |
| Alectinib | ALK |
| Alisertib (MLN8237) | Aurora Kinase |
| ALK inhibitor 1 | ALK |
| ALK inhibitor 2 | ALK |
| ALK-IN-1 | ALK |
| Allitinib tosylate | EGFR |
| Alofanib | FGFR |
| Alpelisib | PI3K |
| Altiratinib | c-Met/HGFR; FLT3; Trk Receptor; VEGFR |
| ALW-II-41-27 | Ephrin Receptor |
| AM-2394 | Glucokinase |
| Amcasertib (BBI503) | Stemness kinase |
| AMG 337 | c-Met |
| AMG 900 | Aurora Kinase |
| AMG 925 (HCl) | CDK; FLT3 |
| AMG-208 | c-Met/HGFR |
| AMG319 | PI3K |
| AMG-337 | c-Met/HGFR |
| AMG-3969 | Glucokinase |
| AMG-458 | c-Met |
| AMG-47a | Src |
| AMG-900 | Aurora Kinase |
| Amlexanox | Immunology & Inflammation related |
| Amuvatinib (MP-470) | c-Kit,FLT3,PDGFR |
| ANA-12 | Trk Receptor |
| Anacardic Acid | Histone Acetyltransferase |
| Anlotinib (AL3818) dihydrochloride | VEGFR |
| AP26113-analog (ALK-IN-1) | ALK,EGFR |
| Apatinib | VEGFR,c-RET |
| Apatinib?mesylate | VEGFR |
| Apigenin | P450 (e.g. CYP17) |
| Apitolisib | mTOR; PI3K |
| APS-2-79 | MEK |
| APY0201 | Interleukin Related; PIKfyve |
| APY29 | IRE1 |
| AR-A014418 | GSK-3 |
| ARN-3236 | Salt-inducible Kinase (SIK) |
| ARQ 531 | Btk |
| AS-252424 | PI3K |
| AS601245 | JNK |
| AS-604850 | PI3K |
| AS-605240 | Autophagy; PI3K |
| Asciminib | Bcr-Abl |
| Asciminib (ABL001) | Bcr-Abl |
| ASP3026 | ALK |
| ASP5878 | FGFR |
| AST 487 | Bcr-Abl; c-Kit; FLT3; VEGFR |
| AST-1306 | EGFR |
| Astragaloside IV | ERK; JNK; MMP |
| AT13148 | Akt,S6 Kinase,ROCK,PKA |
| AT7519 | CDK |
| AT7867 | Akt,S6 Kinase |
| AT9283 | Aurora Kinase,Bcr-Abl,JAK |
| Atuveciclib | CDK |
| Atuveciclib S-Enantiomer | CDK |
| Aurora A inhibitor I | Aurora Kinase |
| Autophinib | Autophagy,PI3K |
| AUZ 454 | CDK |
| AV-412 | EGFR |
| Avapritinib | c-Kit |
| Avitinib (maleate) | EGFR |
| AX-15836 | ERK |
| Axitinib | c-Kit,PDGFR,VEGFR |
| AZ 3146 | Kinesin |
| AZ 628 | Raf |
| AZ 960 | JAK |
| AZ1495 | IRAK |
| AZ191 | DYRK |
| AZ20 | ATM/ATR |
| AZ-23 | Trk Receptor |
| AZ304 | Raf |
| AZ31 | ATM/ATR |
| AZ3146 | Mps1 |
| AZ32 | ATM/ATR |
| AZ5104 | EGFR |
| AZ960 | JAK |
| Azaindole 1 | ROCK |
| AZD 6482 | Autophagy; PI3K |
| AZD0156 | ATM/ATR |
| AZD-0364 | ERK |
| AZD1080 | GSK-3 |
| AZD1152 | Aurora Kinase |
| AZD1208 | Pim |
| AZD1390 | ATM/ATR |
| AZD-1480 | JAK |
| AZD2858 | GSK-3 |
| AZD2932 | PDGFR,VEGFR,FLT3,c-Kit |
| AZD3229 | c-Kit |
| AZD3264 | IκB/IKK |
| AZD3463 | ALK,IGF-1R |
| AZD-3463 | ALK; Autophagy; IGF-1R |
| AZD3759 | EGFR |
| AZD4547 | FGFR |
| AZD4573 | CDK |
| AZD5363 | Akt |
| AZD5438 | CDK |
| AZD-5438 | CDK |
| AZD6482 | PI3K |
| AZD6738 | ATM/ATR |
| AZD7507 | c-Fms |
| AZD7545 | PDHK |
| AZD7762 | Chk |
| AZD-7762 | Checkpoint Kinase (Chk) |
| AZD8055 | mTOR |
| AZD-8055 | Autophagy; mTOR |
| AZD8186 | PI3K |
| AZD8330 | MEK |
| AZD8835 | PI3K |
| AZD-8835 | PI3K |
| AZM475271 | Src |
| Bafetinib (INNO-406) | Bcr-Abl |
| Bakuchiol | Immunology & Inflammation related |
| Barasertib-HQPA | Aurora Kinase |
| Bardoxolone Methyl | IκB/IKK |
| Baricitinib | JAK |
| BAW2881 (NVP-BAW2881) | VEGFR,Raf,c-RET |
| BAY 11-7082 | E2 conjugating,IκB/IKK |
| Bay 11-7085 | IκB/IKK |
| BAY 1217389 | Kinesin,Serine/threonin kinase |
| BAY 1895344 (BAY-1895344) | ATM/ATR |
| Bay 65-1942 (hydrochloride) | IKK |
| BAY1125976 | Akt |
| BAY1217389 | Mps1 |
| BAY-1895344 (hydrochloride) | ATM/ATR |
| BAY-61-3606 | Syk |
| BDP5290 | ROCK |
| BEBT-908 | PI3K |
| Belizatinib | ALK; Trk Receptor |
| Bemcentinib | TAM Receptor |
| Bentamapimod | JNK |
| Berbamine (dihydrochloride) | Bcr-Abl |
| Berberine (chloride hydrate) | Autophagy; Bacterial; ROS; Topoisomerase |
| Berzosertib | ATM/ATR |
| BF738735 | PI4K |
| BFH772 | VEGFR |
| BGG463 | CDK |
| BGT226 (NVP-BGT226) | mTOR,PI3K |
| BI 2536 | PLK |
| BI-4464 | FAK; Ligand for Target Protein |
| BI605906 | IKK |
| BI-78D3 | JNK |
| BI-847325 | MEK,Aurora Kinase |
| BIBF 1202 | VEGFR |
| BIBF0775 | TGF-β Receptor |
| BI-D1870 | S6 Kinase |
| Bikinin | GSK-3 |
| Bimiralisib | mTOR; PI3K |
| Binimetinib | Autophagy; MEK |
| Binimetinib (MEK162, ARRY-162, ARRY-438162) | MEK |
| BIO | GSK-3 |
| BIO-acetoxime | GSK-3 |
| Biochanin A | FAAH |
| Bisindolylmaleimide I | PKC |
| Bisindolylmaleimide I (GF109203X) | PKC |
| Bisindolylmaleimide IX (Ro 31-8220 | |
| Mesylate) | PKC |
| BIX 02188 | MEK |
| BIX 02189 | MEK |
| BIX02188 | ERK; MEK |
| BIX02189 | ERK; MEK |
| BLU-554 (BLU554) | FGFR |
| BLU9931 | FGFR |
| BLZ945 | CSF-1R |
| BMS 777607 | c-Met/HGFR; TAM Receptor |
| BMS-265246 | CDK |
| BMS-345541 | Iκ/IKK |
| BMS-5 | LIM Kinase (LIMK) |
| BMS-509744 | Itk |
| BMS-536924 | IGF-1R |
| BMS-582949 | p38 MAPK |
| BMS-690514 | EGFR; VEGFR |
| BMS-754807 | c-Met,IGF-1R,Trk receptor |
| BMS-777607 | TAM Receptor,c-Met |
| BMS-794833 | c-Met,VEGFR |
| BMS-911543 | JAK |
| BMS-935177 | BTK,Trk receptor,c-RET |
| BMS-986142 | Btk |
| BMS-986195 | Btk |
| BMX-IN-1 | BMX Kinase; Btk |
| BOS-172722 | Mps1 |
| Bosutinib (SKI-606) | Src |
| BPR1J-097 Hydrochloride | FLT3 |
| bpV (HOpic) | PTEN |
| BQR-695 | PI4K |
| B-Raf IN 1 | Raf |
| BRAF inhibitor | Raf |
| B-Raf inhibitor 1 | Raf |
| Brivanib | Autophagy; VEGFR |
| Brivanib (BMS-540215) | FGFR,VEGFR |
| Brivanib Alaninate (BMS-582664) | FGFR,VEGFR |
| BS-181 | CDK |
| BTK IN-1 | Btk |
| Btk inhibitor 1 | Btk |
| BTK inhibitor 1 (Compound 27) | BTK |
| Btk inhibitor 1 (R enantiomer) | Btk |
| Btk inhibitor 2 | Btk |
| Bucladesine (calcium salt) | PKA |
| Bucladesine (sodium salt) | PKA |
| Buparlisib | PI3K |
| Butein | EGFR |
| BX517 | PDK-1 |
| BX795 | PDK-1 |
| BX-795 | IκB/IKK,PDK |
| BX-912 | PDK |
| Ca2+ channel agonist 1 | Calcium Channel; CDK |
| CA-4948 | TLR,IL Receptor |
| Cabozantinib | c-Kit; c-Met/HGFR; FLT3; TAM Receptor; VEGFR |
| Cabozantinib (S-malate) | VEGFR |
| Cabozantinib (XL184, BMS-907351) | c-Met,VEGFR |
| Cabozantinib malate (XL184) | TAM Receptor,VEGFR |
| CAL-130 (Hydrochloride) | PI3K |
| CaMKII-IN-1 | CaMK |
| Canertinib (CI-1033) | EGFR,HER2 |
| Capivasertib | Akt; Autophagy |
| Capmatinib | c-Met/HGFR |
| Casein Kinase II Inhibitor IV | Casein Kinase |
| CAY10505 | PI3K |
| CC-115 | DNA-PK,mTOR |
| CC-223 | mTOR |
| CC-401 (hydrochloride) | JNK |
| CC-671 | CDK |
| CC-90003 | ERK |
| CCG215022 | PKA |
| CCT 137690 | Aurora Kinase |
| CCT020312 | Eukaryotic Initiation Factor (eIF); PERK |
| CCT128930 | Akt |
| CCT129202 | Aurora Kinase |
| CCT137690 | Aurora Kinase |
| CCT196969 | Raf,Src |
| CCT241533 (hydrochloride) | Checkpoint Kinase (Chk) |
| CCT241736 | Aurora Kinase; FLT3 |
| CCT245737 | Chk |
| CCT-251921 | CDK |
| CDK9-IN-1 | CDK; HIV |
| CDK9-IN-2 | CDK |
| CDKI-73 | CDK |
| CDK-IN-2 | CDK |
| Cediranib | Autophagy; PDGFR; VEGFR |
| Cediranib Maleate | VEGFR |
| Centrinone | Polo-like Kinase (PLK) |
| Centrinone-B | Polo-like Kinase (PLK) |
| CEP-28122 (mesylate salt) | ALK |
| CEP-32496 | CSF-1R,Raf |
| CEP-33779 | JAK |
| CEP-37440 | ALK; FAK |
| CEP-40783 | c-Met/HGFR; TAM Receptor |
| Ceralasertib | ATM/ATR |
| Cerdulatinib | JAK; Syk |
| Cerdulatinib (PRT062070, PRT2070) | JAK |
| Ceritinib | ALK; IGF-1R; Insulin Receptor |
| Ceritinib dihydrochloride | ALK; IGF-1R; Insulin Receptor |
| CFI-400945 | PLK |
| CFI-402257 hydrochloride | Mps1 |
| cFMS Receptor Inhibitor II | c-Fms |
| c-Fms-IN-2 | c-Fms |
| CG-806 | Btk; FLT3 |
| CGI1746 | BTK |
| CGI-1746 | Autophagy; Btk |
| CGK 733 | ATM/ATR |
| CGK733 | ATM/ATR |
| CGP 57380 | MNK |
| CGP60474 | PKC; VEGFR |
| CH5132799 | PI3K |
| CH5183284 | FGFR |
| CH5183284 (Debio-1347) | FGFR |
| CH7057288 | Trk Receptor |
| Chelerythrine Chloride | Autophagy; PKC |
| CHIR-124 | Chk |
| CHIR-98014 | GSK-3 |
| CHIR-99021 | Autophagy; GSK-3 |
| CHIR-99021 (CT99021) | GSK-3 |
| Chk2 Inhibitor II (BML-277) | Chk |
| Chloropyramine hydrochloride | FAK; Histamine Receptor; VEGFR |
| CHMFL-BMX-078 | BMX Kinase |
| CHR-6494 | Haspin Kinase |
| Chroman 1 | ROCK |
| Chrysophanic Acid | EGFR,mTOR |
| CHZ868 | JAK |
| CI-1040 | MEK |
| CID 2011756 | Serine/threonin kina |
| CID755673 | Serine/threonin kinase,CaMK |
| CK1-IN-1 | Casein Kinase |
| c-Kit-IN-1 | c-Kit; c-Met/HGFR |
| CL-387785 | EGFR |
| CL-387785 (EKI-785) | EGFR |
| CLK1-IN-1 | CDK |
| c-Met inhibitor 1 | c-Met/HGFR |
| CNX-2006 | EGFR |
| CNX-774 | Btk |
| Cobimetinib | MEK |
| Cobimetinib (GDC-0973, RG7420) | MEK |
| Cobimetinib (hemifumarate) | MEK |
| Cobimetinib (racemate) | MEK |
| Compound 401 | DNA-PK |
| Corynoxeine | ERK1/2 |
| CP21R7 | GSK-3 |
| CP21R7 (CP21) | Wnt/beta-catenin |
| CP-466722 | ATM/ATR |
| CP-673451 | PDGFR |
| CP-724714 | EGFR,HER2 |
| Crenolanib | Autophagy; FLT3; PDGFR |
| Crizotinib | ALK; Autophagy; c-Met/HGFR |
| CRT0066101 | Serine/threonin kinase,CaMK |
| CRT0066101 dihydrochloride | PKD |
| CT7001 hydrochloride | CDK |
| Cucurbitacin E | Autophagy; CDK |
| Cucurbitacin I | JAK; STAT |
| CUDC-101 | EGFR,HDAC,HER2 |
| CUDC-907 | HDAC,PI3K |
| CVT-313 | CDK |
| CX-6258 | Pim |
| Cyasterone | EGFR |
| CYC065 | CDK |
| CYC116 | Aurora Kinase,VEGFR |
| CZ415 | mTOR |
| CZC24832 | PI3K |
| CZC-25146 | LRRK2 |
| CZC-54252 | LRRK2 |
| CZC-8004 | Bcr-Abl |
| D 4476 | Casein Kinase |
| D4476 | Autophagy; Casein Kinase |
| Dabrafenib | Raf |
| Dabrafenib (GSK2118436) | Raf |
| Dabrafenib (Mesylate) | Raf |
| Dabrafenib Mesylate | Raf |
| Dacomitinib | EGFR |
| Dacomitinib (PF299804, PF299) | EGFR |
| Dactolisib (Tosylate) | Autophagy; mTOR; PI3K |
| Danthron | AMPK |
| Danusertib | Aurora Kinase; Autophagy |
| Danusertib (PHA-739358) | Aurora Kinase,Bcr-Abl,c-RET,FGFR |
| Daphnetin | PKA,EGFR,PKC |
| Dasatinib | Bcr-Abl,c-Kit,Src |
| Dasatinib Monohydrate | Src,c-Kit,Bcr-Abl |
| DB07268 | JNK |
| DCC-2618 | c-Kit |
| DCP-LA | PKC |
| DDR1-IN-1 | Others |
| Decernotinib (VX-509) | JAK |
| Defactinib | FAK |
| Degrasyn | Autophagy; Bcr-Abl; Deubiquitinase |
| Deguelin | Akt,PI3K |
| Dehydrocorydaline (chloride) | p38 MAPK |
| Dehydrocostus Lactone | IκB/IKK |
| DEL-22379 | ERK |
| Delcasertib | PKC |
| Delgocitinib | JAK |
| Derazantinib | FGFR |
| Derazantinib(ARQ-087) | FGFR |
| Dicoumarol | PDHK |
| Dihexa | c-Met/HGFR |
| Dihydromyricetin | Autophagy; mTOR |
| Dilmapimod | p38 MAPK |
| Dinaciclib | CDK |
| Dinaciclib (SCH727965) | CDK |
| DMAT | Casein Kinase |
| DMH1 | TGF-beta/Smad |
| DMH-1 | Autophagy; TGF-β Receptor |
| Doramapimod | p38 MAPK; Raf |
| Doramapimod (BIRB 796) | p38 MAPK |
| Dorsomorphin (Compound C) | AMPK |
| Dorsomorphin (dihydrochloride) | AMPK; Autophagy; TGF-β Receptor |
| Dovitinib | c-Kit; FGFR; FLT3; PDGFR; VEGFR |
| Dovitinib (lactate) | FGFR |
| Dovitinib (TKI-258) Dilactic Acid | c-Kit,FGFR,FLT3,PDGFR,VEGFR |
| Dovitinib (TKI258) Lactate | FLT3,c-Kit,FGFR,PDGFR,VEGFR |
| Dovitinib (TKI-258, CHIR-258) | c-Kit,FGFR,FLT3,PDGFR,VEGFR |
| DPH | Bcr-Abl |
| Dubermatinib | TAM Receptor |
| Duvelisib | PI3K |
| Duvelisib (R enantiomer) | PI3K |
| EAI045 | EGFR |
| eCF506 | Src |
| Edicotinib | c-Fms |
| eFT-508 (eFT508) | MNK |
| EG00229 | VEGFR |
| EGFR-IN-3 | EGFR |
| Ellagic acid | Topoisomerase |
| EMD638683 | SGK |
| EMD638683 (R-Form) | SGK |
| EMD638683 (S-Form) | SGK |
| Emodin | Autophagy; Casein Kinase |
| Empesertib | Mps1 |
| Encorafenib | Raf |
| ENMD-2076 | Aurora Kinase,FLT3,VEGFR |
| ENMD-2076 L-(+)-Tartaric acid | Aurora Kinase,FLT3,VEGFR |
| Entospletinib | Syk |
| Entospletinib (GS-9973) | Syk |
| Entrectinib | ALK; Autophagy; ROS; Trk Receptor |
| Entrectinib (RXDX-101) | Trk receptor,ALK |
| Enzastaurin | Autophagy; PKC |
| Enzastaurin (LY317615) | PKC |
| Erdafitinib | FGFR |
| Erdafitinib (JNJ-42756493) | FGFR |
| ERK5-IN-1 | ERK |
| Erlotinib | EGFR |
| ETC-1002 | AMPK; ATP Citrate Lyase |
| ETC-206 | MNK |
| ETP-46321 | PI3K |
| ETP-46464 | ATM/ATR,mTOR |
| Everolimus (RAD001) | mTOR |
| Evobrutinib | Btk |
| EX229 | AMPK |
| Falnidamol | EGFR |
| Fasudil (Hydrochloride) | Autophagy; PKA; ROCK |
| Fedratinib | JAK |
| Fenebrutinib | Btk |
| Ferulic acid | FGFR |
| Ferulic acid methyl ester | p38 MAPK |
| FGF401 | FGFR |
| FGFR4-IN-1 | FGFR |
| FIIN-2 | FGFR |
| FIIN-3 | EGFR; FGFR |
| Filgotinib | JAK |
| Filgotinib (GLPG0634) | JAK |
| Fimepinostat | HDAC; PI3K |
| Fingolimod | LPL Receptor; PAK |
| Fisogatinib | FGFR |
| Flavopiridol | Autophagy; CDK |
| FLLL32 | JAK |
| FLT3-IN-1 | FLT3 |
| FLT3-IN-2 | FLT3 |
| Flufenamic acid | AMPK; Calcium Channel; Chloride Channel; COX; Potassium Channel |
| Flumatinib | Bcr-Abl; c-Kit; PDGFR |
| Flumatinib (mesylate) | Bcr-Abl; c-Kit; PDGFR |
| FM381 | JAK |
| FM-381 | JAK |
| FMK | Ribosomal S6 Kinase (RSK) |
| FN-1501 | CDK; FLT3 |
| Foretinib | c-Met/HGFR; VEGFR |
| Foretinib (GSK1363089) | c-Met,VEGFR |
| Formononetin | Others |
| Fostamatinib (R788) | Syk |
| FR 180204 | ERK |
| FRAX1036 | PAK |
| FRAX486 | PAK |
| FRAX597 | PAK |
| Fruquintinib | VEGFRs |
| Futibatinib | FGFR |
| G-5555 | PAK |
| G-749 | FLT3 |
| Galunisertib | TGF-β Receptor |
| Gambogenic acid | Others |
| Gandotinib | FGFR; FLT3; JAK; VEGFR |
| Gandotinib (LY2784544) | JAK |
| GDC-0077 | PI3K |
| GDC-0084 | PI3K,mTOR |
| GDC-0326 | PI3K |
| GDC-0339 | Pim |
| GDC-0349 | mTOR |
| GDC-0575 (ARRY-575, RG7741) | Chk |
| GDC-0623 | MEK |
| GDC-0834 | Btk |
| GDC-0834 (Racemate) | Btk |
| GDC-0834 (S-enantiomer) | Btk |
| GDC-0879 | Raf |
| Gedatolisib (PF-05212384, PKI-587) | mTOR,PI3K |
| Gefitinib | Autophagy; EGFR |
| Gefitinib (ZD1839) | EGFR |
| Genistein | EGFR,Topoisomerase |
| Gilteritinib (ASP2215) | FLT3,TAM Receptor |
| Ginkgolide C | AMPK; MMP; Sirtuin |
| Ginsenoside Rb1 | Autophagy; IRAK; Mitophagy; Na+/K+ ATPase; NF-κB |
| Ginsenoside Re | Amyloid-β; JNK; NF-κB |
| Glesatinib (hydrochloride) | c-Met/HGFR; TAM Receptor |
| GLPG0634 analog | JAK |
| GNE-0877 | LRRK2 |
| GNE-317 | PI3K |
| GNE-477 | mTOR; PI3K |
| GNE-493 | mTOR; PI3K |
| GNE-7915 | LRRK2 |
| GNE-9605 | LRRK2 |
| GNF-2 | Bcr-Abl |
| GNF-5 | Bcr-Abl |
| GNF-5837 | Trk Receptor |
| GNF-7 | Bcr-Abl |
| Go 6983 | PKC |
| Go6976 | FLT3,JAK,PKC |
| Golvatinib (E7050) | c-Met,VEGFR |
| GSK 3 Inhibitor IX | CDK; GSK-3 |
| GSK 650394 | SGK |
| GSK1059615 | mTOR,PI3K |
| GSK1070916 | Aurora Kinase |
| GSK180736A | ROCK |
| GSK180736A (GSK180736) | ROCK |
| GSK1838705A | ALK,IGF-1R |
| GSK1904529A | IGF-1R |
| GSK2110183 (hydrochloride) | Akt |
| GSK2256098 | FAK |
| GSK2292767 | PI3K |
| GSK2334470 | PDK |
| GSK2578215A | LRRK2 |
| GSK2606414 | PERK |
| GSK2636771 | PI3K |
| GSK2656157 | PERK |
| GSK269962A | ROCK |
| GSK2850163 | IRE1 |
| GSK2982772 | TNF-alpha,NF-κB |
| GSK-3 inhibitor 1 | GSK-3 |
| GSK429286A | ROCK |
| GSK461364 | PLK |
| GSK481 | TNF-alpha |
| GSK'481 | RIP kinase |
| GSK'547 | TNF-alpha |
| GSK583 | NF-κB |
| GSK650394 | Others |
| GSK690693 | Akt |
| GSK-872 | RIP kinase |
| GSK'963 | NF-κB,TNF-alpha |
| Gusacitinib | JAK; Syk |
| GW 441756 | Trk Receptor |
| GW 5074 | Raf |
| GW2580 | CSF-1R |
| GW441756 | Trk receptor |
| GW5074 | Raf |
| GW788388 | TGF-beta/Smad |
| GW843682X | Polo-like Kinase (PLK) |
| GZD824 | Bcr-Abl |
| GZD824 Dimesylate | Bcr-Abl |
| H3B-6527 | FGFR |
| H-89 (dihydrochloride) | Autophagy; PKA |
| HA-100 | Myosin; PKA; PKC |
| Harmine | 5-HT Receptor; DYRK; RAD51 |
| Harmine hydrochloride | DYRK |
| HER2-Inhibitor-1 | EGFR,HER2 |
| Hesperadin | Aurora Kinase |
| HG-10-102-01 | LRRK2 |
| HG-14-10-04 | ALK |
| HG6-64-1 | Raf |
| HG-9-91-01 | Salt-inducible Kinase (SIK) |
| Hispidulin | Pim |
| HMN-214 | PLK |
| Honokiol | Akt,MEK |
| HS-10296 hydrochloride | EGFR |
| HS-1371 | Serine/threonin kina |
| HS-173 | PI3K |
| HTH-01-015 | AMPK |
| hVEGF-IN-1 | VEGFR |
| Hydroxyfasudil | ROCK |
| Ibrutinib | Btk |
| Ibrutinib (PCI-32765) | BTK |
| IC261 | Casein Kinase |
| IC-87114 | PI3K |
| Icotinib | EGFR |
| ID-8 | DYRK |
| Idelalisib | Autophagy; PI3K |
| Idelalisib (CAL-101, GS-1101) | PI3K |
| IITZ-01 | Autophagy; PI3K |
| IKK 16 | IKK; LRRK2 |
| IKK-IN-1 | IKK |
| Ilginatinib | JAK |
| IM-12 | GSK-3 |
| Imatinib | Autophagy; Bcr-Abl; c-Kit; PDGFR |
| Imatinib Mesylate (STI571) | Bcr-Abl,c-Kit,PDGFR |
| IMD 0354 | IκB/IKK |
| IMD-0354 | IKK |
| IMD-0560 | IKK |
| INCB053914 (phosphate) | Pim |
| Indirubin | GSK-3 |
| Indirubin-3'-monoxime | 5-Lipoxygenase; GSK-3 |
| Infigratinib | FGFR |
| Ingenol | PKC |
| INH14 | IKK |
| IPA-3 | PAK |
| lpatasertib | Akt |
| IPI-3063 | PI3K |
| IPI549 | PI3K |
| IPI-549 | PI3K |
| IQ-1S (free acid) | JNK |
| IRAK inhibitor 1 | IRAK |
| IRAK inhibitor 2 | IRAK |
| IRAK inhibitor 4 (trans) | IRAK |
| IRAK inhibitor 6 | IRAK |
| IRAK-1-4 Inhibitor I | IRAK |
| IRAK4-IN-1 | IRAK |
| Irbinitinib (ARRY-380, ONT-380) | HER2 |
| ISCK03 | c-Kit |
| Isorhamnetin | MEK; PI3K |
| Isorhamnetin 3-O-neohesperoside | Others |
| Isovitexin | JNK; NF-κB |
| ISRIB (trans-isomer) | PERK |
| Itacitinib | JAK |
| ITD-1 | TGF-β Receptor |
| ITX5061 | p38 MAPK |
| JAK3-IN-1 | JAK |
| JANEX-1 | JAK |
| JH-II-127 | LRRK2 |
| JH-VIII-157-02 | ALK |
| JI-101 | Ephrin Receptor; PDGFR; VEGFR |
| JNJ-38877605 | c-Met |
| JNJ-38877618 | c-Met/HGFR |
| JNJ-47117096 hydrochloride | FLT3; MELK |
| JNJ-7706621 | Aurora Kinase,CDK |
| JNK Inhibitor IX | JNK |
| JNK-IN-7 | JNK |
| JNK-IN-8 | JNK |
| K02288 | TGF-beta/Smad |
| K03861 | CDK |
| K145 (hydrochloride) | SPHK |
| kb NB 142-70 | PKD |
| KD025 (SLx-2119) | ROCK |
| KDU691 | PI4K |
| Kenpaullone | CDK |
| Ki20227 | c-Fms |
| Ki8751 | c-Kit,PDGFR,VEGFR |
| kira6 | Others |
| KN-62 | CaMK |
| KN-92 (hydrochloride) | CaMK |
| KN-93 | CaMK |
| KN-93 Phosphate | CaMK |
| KPT-9274 | NAMPT,PAK |
| KRN 633 | VEGFR |
| KU-0063794 | mTOR |
| KU-55933 | ATM/ATR; Autophagy |
| KU-57788 | CRISPR/Cas9; DNA-PK |
| KU-60019 | ATM/ATR |
| KW-2449 | Aurora Kinase,Bcr-Abl,FLT3 |
| KX1-004 | Src |
| KX2-391 | Src |
| L-779450 | Autophagy; Raf |
| Lapatinib | EGFR,HER2 |
| Larotrectinib (LOXO-101) sulfate | Trk receptor |
| Larotrectinib sulfate | Trk Receptor |
| Lazertinib | EGFR |
| Lazertinib (YH25448,GNS-1480) | EGFR |
| Lck Inhibitor | Src |
| Lck inhibitor 2 | Src |
| LDC000067 | CDK |
| LDC1267 | TAM Receptor |
| LDC4297 | CDK |
| LDN-193189 2HCl | TGF-beta/Smad |
| LDN-212854 | TGF-β Receptor |
| LDN-214117 | TGF-beta/Smad |
| Leflunomide | Dehydrogenase |
| Leniolisib | PI3K |
| Lenvatinib | VEGFR |
| Lerociclib dihydrochloride | CDK |
| LFM-A13 | BTK |
| Lifirafenib | EGFR; Raf |
| Linifanib | Autophagy; FLT3; PDGFR; VEGFR |
| Linsitinib | IGF-1R; Insulin Receptor |
| LJH685 | S6 Kinase |
| LJI308 | S6 Kinase |
| L-Leucine | mTOR |
| LM22A-4 | Trk Receptor |
| LM22B-10 | Akt; ERK; Trk Receptor |
| Longdaysin | Casein Kinase; ERK |
| Lonidamine | Hexokinase |
| Lorlatinib | ALK |
| Lorlatinib?(PF-6463922) | ALK |
| Losmapimod | Autophagy; p38 MAPK |
| Losmapimod (GW856553X) | p38 MAPK |
| Loureirin B | ERK; JNK; PAI-1; Potassium Channel |
| LRRK2 inhibitor 1 | LRRK2 |
| LRRK2-IN-1 | LRRK2 |
| LSKL, Inhibitor of Thrombospondin (TSP-1) | TGF-β Receptor |
| LTURM34 | DNA-PK |
| Lucitanib | FGFR; VEGFR |
| Lupeol | Immunology & Inflammation related |
| LX2343 | Amyloid-β; Autophagy; Beta-secretase; PI3K |
| LXH254 | Raf |
| LXS196 | PKC |
| LY2090314 | GSK-3 |
| LY2109761 | TGF-beta/Smad |
| LY2409881 | IκB/IKK |
| LY2584702 | S6 Kinase |
| LY2584702 Tosylate | S6 Kinase |
| LY2608204 | Glucokinase |
| LY2857785 | CDK |
| LY2874455 | FGFR,VEGFR |
| LY294002 | Autophagy,Pl3K |
| LY3009120 | Raf |
| LY3023414 | mTOR,PI3K,DNA-PK |
| LY3177833 | CDK |
| LY3200882 | TGF-β Receptor |
| LY3214996 | ERK |
| LY3295668 | Aurora Kinase |
| LY364947 | TGF-beta/Smad |
| LY-364947 | TGF-β Receptor |
| LYN-1604 hydrochloride | ULK |
| Magnolin | ERK1 |
| Masitinib | c-Kit; PDGFR |
| MBQ-167 | CDK; Ras |
| MC180295 | CDK |
| MCB-613 | Src |
| MEK inhibitor | MEK |
| MELK-8a (hydrochloride) | MELK |
| Merestinib | c-Met/HGFR |
| Mesalamine | IκB/IKK, Immunology & Inflammation related |
| Metadoxine | PKA |
| Metformin (hydrochloride) | AMPK; Autophagy; Mitophagy |
| Methylthiouracil | ERK; Interleukin Related; NF-κB; TNF Receptor |
| MGCD-265 analog | c-Met/HGFR; VEGFR |
| MHP | SPHK |
| MHY1485 | Autophagy; mTOR |
| Midostaurin | PKC |
| Milciclib (PHA-848125) | CDK |
| Miltefosine | Akt |
| Miransertib | Akt |
| Mirin | ATM/ATR |
| Mirk-IN-1 | DYRK |
| Mitoxantrone | PKC; Topoisomerase |
| MK 2206 (dihydrochloride) | Akt; Autophagy |
| MK-2461 | c-Met,FGFR,PDGFR |
| MK2-IN-1 (hydrochloride) | MAPKAPK2 (MK2) |
| MK-3903 | AMPK |
| MK-5108 | Aurora Kinase |
| MK-8033 | c-Met/HGFR |
| MK8722 | AMPK |
| MK-8745 | Aurora Kinase |
| MK-8776 (SCH 900776) | CDK,Chk |
| MKC3946 | IRE1 |
| MKC8866 | IRE1 |
| MKC9989 | IRE1 |
| ML167 | CDK |
| ML347 | TGF-beta/Smad,ALK |
| ML-7 HCl | Serine/threonin kinase |
| MLi-2 | LRRK2 |
| MLN0905 | PLK |
| MLN120B | IKK |
| MLN2480 | Raf |
| MLN8054 | Aurora Kinase |
| MNS | Src; Syk |
| MNS (3,4-Methylenedioxy-β-nitrostyrene, MDBN) | Tyrosinase,p97,Syk,Src |
| Momelotinib | Autophagy; JAK |
| Motesanib | c-Kit; VEGFR |
| MP7 | PDK-1 |
| MP-A08 | SPHK |
| MPI-0479605 | Kinesin |
| Mps1-IN-1 | Mps1 |
| Mps1-IN-2 | Mps1; Polo-like Kinase (PLK) |
| MRT67307 HCl | IkB/IKK |
| MRT68921 (hydrochloride) | ULK |
| MRX-2843 | FLT3 |
| MSC2530818 | CDK |
| MSDC 0160 | Insulin Receptor |
| mTOR inhibitor-3 | mTOR |
| MTX-211 | EGFR; PI3K |
| Mubritinib | EGFR |
| Mutated EGFR-IN-1 | EGFR |
| Myricetin | MEK |
| NAMI-A | FAK |
| Naquotinib(ASP8273) | EGFR |
| Narciclasine | ROCK |
| Nazartinib | EGFR |
| Nazartinib (EGF816, NVS-816) | EGFR |
| NCB-0846 | Wnt/beta-catenin |
| Nec-1s (7-Cl-O-Nec1) | TNF-alpha |
| Necrostatin-1 | Autophagy; RIP kinase |
| Necrosulfonamide | Others |
| Nedisertib | DNA-PK |
| Neflamapimod | p38 MAPK |
| Nemiralisib | PI3K |
| Neohesperidin dihydrochalcone | ROS |
| Neratinib (HKI-272) | EGFR,HER2 |
| NG 52 | CDK |
| NH125 | CaMK |
| Nilotinib | Autophagy; Bcr-Abl |
| Nilotinib (AMN-107) | Bcr-Abl |
| Ningetinib | c-Met/HGFR; TAM Receptor; VEGFR |
| Nintedanib | FGFR; PDGFR; VEGFR |
| NMS-P937 (NMS1286937) | PLK |
| Nocodazole | Autophagy, Microtubule Associated |
| Norcantharidin | EGFR,c-Met |
| Notoginsenoside R1 | Others |
| NPS-1034 | c-Met,TAM Receptor |
| NQDI-1 | ASK |
| NSC 228155 | EGFR; Epigenetic Reader Domain; Histone Acetyltransferase |
| NSC 42834 | JAK |
| NSC12 | FGFR |
| NSC781406 | mTOR; PI3K |
| NT157 | IGF-1R |
| NU 7026 | DNA-PK |
| NU2058 | CDK |
| NU6027 | CDK |
| NU6300 | CDK |
| NU7026 | DNA-PK |
| NU7441 (KU-57788) | DNA-PK,PI3K |
| NVP-2 | CDK |
| NVP-ACC789 | PDGFR; VEGFR |
| NVP-ADW742 | IGF-1R |
| NVP-BAW2881 | VEGFR |
| NVP-BHG712 | Bcr-Abl,Ephrin receptor,Raf,Src |
| NVP-BHG712 isomer | Ephrin Receptor |
| NVP-BSK805 2HCl | JAK |
| NVP-BVU972 | c-Met |
| NVP-LCQ195 | CDK |
| NVP-TAE 226 | FAK; Pyk2 |
| NVP-TAE 684 | ALK |
| NVS-PAK1-1 | PAK |
| Oclacitinib (maleate) | JAK |
| Oglufanide | VEGFR |
| Olmutinib | EGFR |
| Omipalisib | mTOR; PI3K |
| Omtriptolide | ERK |
| ON123300 | CDK |
| ONO-4059 (GS-4059) hydrochloride | BTK |
| Orantinib (TSU-68, SU6668) | PDGFR |
| Oridonin | Akt |
| OSI-027 | mTOR |
| OSI-420 | EGFR |
| OSI-930 | c-Kit,CSF-1R,VEGFR |
| Osimertinib | EGFR |
| OSU-03012 (AR-12) | PDK |
| OTS514 hydrochloride | TOPK |
| OTS964 | TOPK |
| OTSSP167 (hydrochloride) | MELK |
| P276-00 | CDK |
| p38α inhibitor 1 | p38 MAPK |
| p38-α MAPK-IN-1 | p38 MAPK |
| Pacritinib | FLT3; JAK |
| Palbociclib (hydrochloride) | CDK |
| Palbociclib (isethionate) | CDK |
| Palomid 529 | mTOR |
| Palomid 529 (P529) | mTOR |
| Pamapimod | p38 MAPK |
| Parsaclisib | PI3K |
| Pazopanib | c-Kit,PDGFR,VEGFR |
| PCI 29732 | Btk |
| PCI-33380 | Btk |
| PD 169316 | Autophagy; p38 MAPK |
| PD0166285 | Wee1 |
| PD0325901 | MEK |
| PD153035 | EGFR |
| PD158780 | EGFR |
| PD-166866 | FGFR |
| PD168393 | EGFR |
| PD173074 | FGFR,VEGFR |
| PD173955 | Bcr-Abl |
| PD184352 (CI-1040) | MEK |
| PD318088 | MEK |
| PD98059 | MEK |
| Peficitinib | JAK |
| Pelitinib | EGFR; Src |
| Pelitinib (EKB-569) | EGFR |
| Pemigatinib | FGFR |
| Perifosine (KRX-0401) | Akt |
| Pexidartinib | c-Fms; c-Kit |
| Pexmetinib (ARRY-614) | p38 MAPK,Tie-2 |
| PF-00562271 Besylate | FAK |
| PF-03814735 | Aurora Kinase; VEGFR |
| PF-04217903 | c-Met |
| PF-04217903 (methanesulfonate) | c-Met/HGFR |
| PF-04691502 | Akt,mTOR,PI3K |
| PF-04965842 | JAK |
| PF-05231023 | FGFR |
| PF-06273340 | Trk receptor |
| PF-06409577 | AMPK |
| PF-06447475 | LRRK2 |
| PF-06459988 | EGFR |
| PF06650833 | IRAK |
| PF-06651600 | JAK |
| PF-06700841 (P-Tosylate) | JAK |
| PF-3758309 | PAK |
| PF-431396 | FAK |
| PF-4708671 | S6 Kinase |
| PF-477736 | Chk |
| PF-4800567 | Casein Kinase |
| PF-4989216 | PI3K |
| PF-543 (Citrate) | SPHK |
| PF-562271 | FAK |
| PF-573228 | FAK |
| PFK15 | Autophagy |
| PFK158 | Autophagy |
| PH-797804 | p38 MAPK |
| PHA-665752 | c-Met |
| PHA-680632 | Aurora Kinase |
| PHA-767491 | CDK |
| PHA-793887 | CDK |
| Phenformin (hydrochloride) | AMPK |
| Phorbol 12-myristate 13-acetate | PKC; SPHK |
| PHT-427 | Akt,PDK |
| PI-103 | Autophagy,DNA-PK,mTOR,PI3K |
| PI-103 (Hydrochloride) | DNA-PK; mTOR; PI3K |
| PI-3065 | PI3K |
| PI3K-IN-1 | PI3K |
| PI3Kδ-IN-2 | PI3K |
| PI4KIII beta inhibitor 3 | PI4K |
| Piceatannol | Syk |
| Picfeltarraenin IA | AChE |
| Picropodophyllin | IGF-1R |
| Pictilisib (GDC-0941) | PI3K |
| PIK-293 | PI3K |
| PIK-294 | PI3K |
| PIK-75 | DNA-PK; PI3K |
| PIK-75 HCl | DNA-PK,PI3K |
| PIK-93 | PI3K |
| PIK-III | Autophagy,PI3K |
| Pilaralisib | PI3K |
| Pilaralisib analogue | PI3K |
| Pim1/AKK1-IN-1 | Pim |
| PIM-447 (dihydrochloride) | Pim |
| Pimasertib | MEK |
| Pitavastatin Calcium | HMG-CoA Reductase |
| PKC-IN-1 | PKC |
| PKC-theta inhibitor | PKC |
| PKM2 inhibitor(compound 3k) | PKM |
| Pluripotin | ERK; Ribosomal S6 Kinase (RSK) |
| PLX-4720 | Raf |
| PLX647 | c-Fms; c-Kit |
| PLX7904 | Raf |
| PLX8394 | Raf |
| PND-1186 | FAK |
| PND-1186 (VS-4718) | FAK |
| Poloxime | Polo-like Kinase (PLK) |
| Poloxin | Polo-like Kinase (PLK) |
| Ponatinib (AP24534) | Bcr-Abl,FGFR,PDGFR,VEGFR |
| Poziotinib (HM781-36B) | HER2,EGFR |
| PP1 | Src |
| PP121 | DNA-PK,mTOR,PDGFR,Src,VEGFR,Bcr-Abl |
| PP2 | Src |
| PQ 401 | IGF-1R |
| PQR620 | mTOR |
| Prexasertib | Checkpoint Kinase (Chk) |
| PRN1008 | Btk |
| PRN1371 | FGFR |
| PRN694 | Itk |
| PROTAC CDK9 Degrader-1 | CDK; PROTAC |
| Protein kinase inhibitors 1 hydrochloride | DYRK |
| PRT-060318 | Syk |
| PRT062607 (Hydrochloride) | Syk |
| PS-1145 | IκB/IKK |
| Psoralidin | Estrogen/progestogen Receptor |
| Purvalanol A | CDK |
| Purvalanol B | CDK |
| PYR-41 | E1 Activating |
| Pyridone 6 | JAK |
| Pyrotinib dimaleate | EGFR |
| Quercetin | Src,Sirtuin, PKC,PI3K |
| Quizartinib (AC220) | FLT3 |
| R112 | Syk |
| R1487 (Hydrochloride) | p38 MAPK |
| R1530 | VEGFR |
| R-268712 | TGF-β Receptor |
| R406 | FLT3,Syk |
| R406 (free base) | Syk |
| R547 | CDK |
| R788 (Fostamatinib) Disodium | Syk |
| Rabusertib (LY2603618) | Chk |
| Radotinib | Bcr-Abl |
| RAF265 | Autophagy; Raf; VEGFR |
| RAF265 (CHIR-265) | Raf,VEGFR |
| RAF709 | Raf |
| Ralimetinib (LY2228820) | p38 MAPK |
| Rapamycin (Sirolimus) | Autophagy,mTOR |
| Ravoxertinib | ERK |
| Rebastinib | Bcr-Abl; FLT3; Src |
| Refametinib | MEK |
| Refametinib (RDEA119, Bay 86-9766) | MEK |
| Regorafenib | Autophagy; PDGFR; Raf; VEGFR |
| Repotrectinib | ALK; ROS; Trk Receptor |
| RepSox | TGF-beta/Smad |
| Resveratrol | Autophagy; IKK; Mitophagy; Sirtuin |
| Reversine | Adenosine Receptor,Aurora Kinase |
| RG13022 | EGFR |
| RG14620 | EGFR |
| RGB-286638 (free base) | CDK; GSK-3; JAK; MEK |
| Ribociclib | CDK |
| Ridaforolimus (Deforolimus, MK-8669) | mTOR |
| Rigosertib (ON-01910) | PLK |
| Rigosertib (sodium) | Polo-like Kinase (PLK) |
| Rimacalib | CaMK |
| RIP2 kinase inhibitor 1 | RIP kinase |
| RIP2 kinase inhibitor 2 | RIP kinase |
| RIPA-56 | RIP kinase |
| Ripasudil | ROCK |
| Ripretinib | c-Kit; PDGFR |
| RK-24466 | Src |
| RKI-1447 | ROCK |
| RN486 | Btk |
| Ro 28-1675 | Glucokinase |
| Ro 5126766 | MEK; Raf |
| Ro3280 | PLK |
| Ro-3306 | CDK |
| RO4987655 | MEK |
| RO9021 | Syk |
| Roblitinib | FGFR |
| Rociletinib | EGFR |
| Rociletinib (CO-1686, AVL-301) | EGFR |
| Rociletinib hydrobromide | EGFR |
| Rogaratinib | FGFR |
| Roscovitine (Seliciclib,CYC202) | CDK |
| Rosmarinic acid | IκB/IKK |
| Ruboxistaurin (LY333531 HCl) | PKC |
| Ruxolitinib | Autophagy; JAK; Mitophagy |
| Ruxolitinib (phosphate) | Autophagy; JAK; Mitophagy |
| Ruxolitinib (S enantiomer) | Autophagy; JAK |
| RXDX-106 (CEP-40783) | TAM Receptor |
| S49076 | c-Met,FGFR,TAM Receptor |
| SAFit2 | Akt |
| Salidroside | mTOR |
| Salubrinal | PERK |
| Sapanisertib | Autophagy; mTOR |
| Sapitinib | EGFR |
| SAR-020106 | Chk |
| SAR125844 | c-Met |
| SAR131675 | VEGFR |
| SAR-20347 | JAK |
| SAR-260301 | PI3K |
| SAR405 | Autophagy; PI3K |
| SAR407899 | ROCK |
| Saracatinib | Autophagy; Src |
| Saracatinib (AZD0530) | Src |
| Savolitinib | c-Met/HGFR |
| Savolitinib(AZD6094, HMPL-504) | c-Met |
| SB 202190 | Autophagy; p38 MAPK |
| SB 203580 | Autophagy; Mitophagy; p38 MAPK |
| SB 203580 (hydrochloride) | Autophagy; Mitophagy; p38 MAPK |
| SB 239063 | p38 MAPK |
| SB 242235 | p38 MAPK |
| SB 415286 | GSK-3 |
| SB 525334 | TGF-β Receptor |
| SB1317 | CDK; FLT3; JAK |
| SB202190 (FHPI) | p38 MAPK |
| SB203580 | p38 MAPK |
| SB216763 | GSK-3 |
| SB239063 | p38 MAPK |
| SB415286 | GSK-3 |
| SB431542 | TGF-beta/Smad |
| SB-431542 | TGF-β Receptor |
| SB505124 | TGF-beta/Smad |
| SB-505124 | TGF-β Receptor |
| SB525334 | TGF-beta/Smad |
| SB590885 | Raf |
| SB-590885 | Raf |
| SBE 13 HCl | PLK |
| SBI-0206965 | Autophagy |
| SC-514 | IκB/IKK |
| SC66 | Akt |
| SC79 | Akt |
| SCH-1473759 (hydrochloride) | Aurora Kinase |
| SCH772984 | ERK |
| SCH900776 | Checkpoint Kinase (Chk) |
| Schisandrin B (Sch B) | ATM/ATR,P-gp |
| Scopoletin | Immunology & Inflammation related |
| SCR-1481B1 | c-Met/HGFR; VEGFR |
| Scutellarein | Autophagy; Src |
| Scutellarin | Akt; STAT |
| SD 0006 | p38 MAPK |
| SD-208 | TGF-beta/Smad |
| SEL120-34A (monohydrochloride) | CDK |
| Seletalisib | PI3K |
| Seletalisib (UCB-5857) | PI3K |
| Seliciclib | CDK |
| Selitrectinib | Trk Receptor |
| Selonsertib (GS-4997) | ASK |
| Selumetinib | MEK |
| Selumetinib (AZD6244) | MEK |
| Semaxanib (SU5416) | VEGFR |
| Semaxinib | VEGFR |
| Senexin A | CDK |
| Sennoside B | PDGFR |
| Serabelisib | PI3K |
| Serabelisib (INK-1117,MLN-1117,TAK-117) | PI3K |
| SF1670 | PTEN |
| SF2523 | PI3K,DNA-PK,Epigenetic Reader Domain,mTOR |
| SGI-1776 | Autophagy; Pim |
| SGI-1776 free base | Pim |
| SGI-7079 | VEGFR |
| SGX-523 | c-Met |
| Silmitasertib | Autophagy; Casein Kinase |
| Simurosertib | CDK |
| | c-Kit; Discoidin Domain Receptor; FLT3; Trk Receptor; |
| Sitravatinib | VEGFR |
| Sitravatinib (MGCD516) | Ephrin receptor,c-Kit,TAM Receptor,VEGFR,Trk receptor |
| SJ000291942 | TGF-β Receptor |
| SK1-IN-1 | SPHK |
| Skatole | Aryl Hydrocarbon Receptor; p38 MAPK |
| Skepinone-L | p38 MAPK |
| SKF-86002 | p38 MAPK |
| SKI II | S1P Receptor |
| SKLB1002 | VEGFR |
| SKLB4771 | FLT3 |
| SL327 | MEK |
| SL-327 | MEK |
| SLV-2436 | MNK |
| SLx-2119 | ROCK |
| SM 16 | TGF-β Receptor |
| SMI-16a | Pim |
| SMI-4a | Pim |
| SNS-032 | CDK |
| SNS-032 (BMS-387032) | CDK |
| SNS-314 | Aurora Kinase |
| SNS-314 Mesylate | Aurora Kinase |
| Sodium dichloroacetate (DCA) | Dehydrogenase |
| Sodium Monofluorophosphate | phosphatase |
| Solanesol (Nonaisoprenol) | FAK |
| Solcitinib | JAK |
| Sorafenib | Raf |
| Sorafenib Tosylate | PDGFR,Raf,VEGFR |
| Sotrastaurin | PKC |
| SP600125 | JNK |
| Spebrutinib | Btk |
| SPHINX31 | Serine/threonin kina |
| SR-3029 | Casein Kinase |
| SR-3306 | JNK |
| SR-3677 | Autophagy; ROCK |
| Src Inhibitor 1 | Src |
| SRPIN340 | SRPK |
| S-Ruxolitinib (INCB018424) | JAK |
| SSR128129E | FGFR |
| Staurosporine | PKA; PKC |
| STF-083010 | IRE1 |
| STO-609 | CaMK |
| SU 5402 | FGFR; PDGFR; VEGFR |
| SU11274 | c-Met |
| SU14813 | c-Kit; PDGFR; VEGFR |
| SU14813 (maleate) | c-Kit; PDGFR; VEGFR |
| SU1498 | VEGFR |
| SU5402 | FGFR,VEGFR |
| SU5408 | VEGFR |
| SU6656 | Src |
| SU9516 | CDK |
| Sulfatinib | FGFR; VEGFR |
| SUN11602 | FGFR |
| Sunitinib | PDGFR,c-Kit,VEGFR |
| Sunitinib Malate | c-Kit,PDGFR,VEGFR |
| T56-LIMKi | LIM Kinase (LIMK) |
| TA-01 | Casein Kinase; p38 MAPK |
| TA-02 | p38 MAPK |
| TAE226 (NVP-TAE226) | FAK |
| TAE684 (NVP-TAE684) | ALK |
| TAK-285 | EGFR,HER2 |
| TAK-580 | Raf |
| TAK-593 | PDGFR; VEGFR |
| TAK-632 | Raf |
| TAK-659 | Syk,FLT3 |
| TAK-715 | p38 MAPK |
| TAK-733 | MEK |
| TAK-901 | Aurora Kinase |
| TAK-960 | Polo-like Kinase (PLK) |
| Takinib | IL Receptor |
| Talmapimod | p38 MAPK |
| Tandutinib | FLT3 |
| Tandutinib (MLN518) | FLT3 |
| Tanzisertib | JNK |
| Tanzisertib(CC-930) | JNK |
| tarloxotinib bromide | EGFR |
| TAS-115 mesylate | c-Met/HGFR; VEGFR |
| TAS-301 | PKC |
| TAS6417 | EGFR |
| Taselisib | PI3K |
| Tat-NR2B9c | p38 MAPK |
| Tat-NR2B9c (TFA) | p38 MAPK |
| Tauroursodeoxycholate (Sodium) | Caspase; ERK |
| Tauroursodeoxycholate dihydrate | Caspase; ERK |
| Taxifolin (Dihydroquercetin) | VEGFR |
| TBB | Casein Kinase |
| TBK1/IKKε-IN-2 | IKK |
| TC13172 | Mixed Lineage Kinase |
| TC-DAPK 6 | DAPK |
| TCS 359 | FLT3 |
| TCS JNK 5a | JNK |
| TCS PIM-1 1 | Pim |
| TCS-PIM-1-4a | Pim |
| TDZD-8 | GSK-3 |
| Telatinib | c-Kit,PDGFR,VEGFR |
| Temsirolimus (CCI-779, NSC 683864) | mTOR |
| Tenalisib | PI3K |
| Tenalisib (RP6530) | PI3K |
| Tepotinib | Autophagy; c-Met/HGFR |
| Tepotinib (EMD 1214063) | c-Met |
| TG 100572 (Hydrochloride) | FGFR; PDGFR; Src; VEGFR |
| TG003 | CDK |
| TG100-115 | PI3K |
| TG100713 | PI3K |
| TG101209 | c-RET,FLT3,JAK |
| TGX-221 | PI3K |
| Theliatinib (HMPL-309) | EGFR |
| Thiazovivin | ROCK |
| THZ1 | CDK |
| THZ1-R | CDK |
| THZ2 | CDK |
| THZ531 | CDK |
| TIC10 | Akt |
| TIC10 Analogue | Akt |
| Tideglusib | GSK-3 |
| Tie2 kinase inhibitor | Tie-2 |
| Tirabrutinib | Btk |
| Tirbanibulin (Mesylate) | Microtubule/Tubulin; Src |
| Tivantinib | c-Met/HGFR |
| Tivantinib (ARQ 197) | c-Met |
| Tivozanib | VEGFR |
| Tivozanib (AV-951) | c-Kit,PDGFR,VEGFR |
| Toceranib phosphate | PDGFRβ |
| Tofacitinib | JAK |
| Tofacitinib (CP-690550,Tasocitinib) | JAK |
| Tolimidone | Src |
| Tomivosertib | MNK |
| Torin 1 | Autophagy,mTOR |
| Torin 2 | ATM/ATR,mTOR |
| Torkinib | Autophagy; Mitophagy; mTOR |
| Tozasertib (VX-680, MK-0457) | Aurora Kinase |
| TP0427736 HCl | ALK |
| TP-0903 | TAM Receptor |
| TP-3654 | Pim |
| TPCA-1 | IkB/IKK |
| TPPB | PKC |
| TPX-0005 | Src,ALK |
| Trametinib | MEK |
| trans-Zeatin | ERK; MEK |
| Trapidil | PDGFR |
| Triciribine | Akt |
| TTP 22 | Casein Kinase |
| Tucatinib | EGFR |
| TWS119 | GSK-3 |
| TyK2-IN-2 | JAK |
| Tyk2-IN-4 | JAK |
| Tyrosine kinase inhibitor | c-Met/HGFR |
| Tyrosine kinase-IN-1 | FGFR; PDGFR; VEGFR |
| Tyrphostin 23 | EGFR |
| Tyrphostin 9 | PDGFR,EGFR |
| Tyrphostin A9 | VEGFR |
| Tyrphostin AG 1296 | c-Kit,PDGFR |
| Tyrphostin AG 528 | EGFR |
| Tyrphostin AG 879 | HER2 |
| U0126 | Autophagy; MEK; Mitophagy |
| U0126-EtOH | MEK |
| UCB9608 | PI4K |
| UK-371804 HCl | Serine Protease |
| Ulixertinib | ERK |
| ULK-101 | ULK |
| UM-164 | Src,p38 MAPK |
| Umbralisib | PI3K |
| Umbralisib R-enantiomer | PI3K |
| UNC2025 | TAM Receptor,FLT3 |
| UNC2881 | TAM Receptor |
| Upadacitinib | JAK |
| Uprosertib | Akt |
| URMC-099 | LRRK2 |
| Vactosertib | TGF-β Receptor |
| Vactosertib (Hydrochloride) | TGF-β Receptor |
| Valrubicin | PKC |
| Vandetanib | Autophagy; VEGFR |
| Varlitinib | EGFR |
| Vatalanib (PTK787) 2HCl | VEGFR |
| VE-821 | ATM/ATR |
| VE-822 | ATM/ATR |
| Vecabrutinib | Btk; Itk |
| Vemurafenib | Autophagy; Raf |
| VER-246608 | PDHK |
| Verbascoside | Immunology & Inflammation related |
| Vistusertib | Autophagy; mTOR |
| Volasertib (BI 6727) | PLK |
| VO-Ohpic trihydrate | PTEN |
| Voxtalisib | mTOR; PI3K |
| VPS34 inhibitor 1 (Compound 19, PIK-III | |
| analogue) | PI3K |
| Vps34-IN-1 | PI3K |
| Vps34-IN-2 | PI3K |
| Vps34-PIK-III | Autophagy; PI3K |
| VS-5584 | mTOR; PI3K |
| VS-5584 (SB2343) | PI3K |
| VTX-27 | PKC |
| VX-11e | ERK |
| VX-702 | p38 MAPK |
| VX-745 | p38 MAPK |
| WAY-600 | mTOR |
| Wedelolactone | NF-κB |
| WEHI-345 | RIP kinase |
| WH-4-023 | Src |
| WHI-P154 | EGFR; JAK |
| WHI-P180 | EGFR; VEGFR |
| WHI-P97 | JAK |
| WNK463 | Serine/threonin kinase |
| Wogonin | CDK,Transferase |
| Wortmannin | ATM/ATR; DNA-PK; PI3K; Polo-like Kinase (PLK) |
| WP1066 | JAK; STAT |
| WYE-125132 (WYE-132) | mTOR |
| WYE-132 | mTOR |
| WYE-354 | mTOR |
| WZ3146 | EGFR |
| WZ-3146 | EGFR |
| WZ4002 | EGFR |
| WZ4003 | AMPK |
| WZ8040 | EGFR |
| X-376 | ALK; c-Met/HGFR |
| XL019 | JAK |
| XL147 analogue | PI3K |
| XL228 | Aurora Kinase; Bcr-Abl; IGF-1R; Src |
| XL388 | mTOR |
| XL413 (BMS-863233) | CDK |
| XMD16-5 | ACK |
| XMD17-109 | ERK |
| XMD8-87 | ACK |
| XMD8-92 | ERK |
| Y15 | FAK |
| Y-27632 | ROCK |
| Y-33075 | ROCK |
| Y-39983 HCl | ROCK |
| YKL-05-099 | Salt-inducible Kinase (SIK) |
| YLF-466D | AMPK |
| YM-201636 | Autophagy; PI3K; PIKfyve |
| YU238259 | DNA-PK |
| Zanubrutinib | Btk |
| ZD-4190 | EGFR; VEGFR |
| ZINC00881524 | ROCK |
| ZINC00881524 (ROCK inhibitor) | ROCK |
| ZLN024 (hydrochloride) | AMPK |
| ZM 306416 | VEGFR |
| ZM 323881 HCl | VEGFR |
| ZM 336372 | Raf |
| ZM 39923 HCl | JAK |
| ZM 447439 | Aurora Kinase |
| ZM39923 (hydrochloride) | JAK |
| ZM-447439 | Aurora Kinase |
| Zotarolimus(ABT-578) | mTOR |
| ZSTK474 | PI3K |

### REFERENCES

[1] Bhola NE, Jansen VM, Bafna S, Giltnane JM, Balko JM, et al. (2014) Kinome-wide functional screen identifies role of PLK1 in hormone-independent, ER-positive breast cancer. *Cancer Research - Therapeutics, Targets, and Chemical Biology.* DOI: 10.1158/0008-5472.CAN-14-2475.
[2] Maire V, Némati F, Richardson M, Vincent-Salomon A, Tesson B, et al. (2012) *Cancer Research - Therapeutics, Targets, and Chemical Biology.* DOI: 10.1158/0008-5472.CAN-12-2633.

## Claims

1. An in-vitro method of identifying a protein kinase inhibitor for normalizing post-transcriptional regulation which inhibitor is suitable to be used in a targeted cancer therapy, said method comprising the following steps:
a. transfecting cancer cells or a tissue of a cancer patient with at least one expression vector comprising:
i. a promoter region comprising a non-inducible constitutively active ribosomal protein gene promoter, preferably a promoter that comprises a modified promoter of the human RPS30 gene that has the nucleic acid sequence of SEQ ID NO:3 (RPS30M1) or SEQ ID NO:4 (RPS30M-truncated),
ii. a reporter gene; and
iii. a 3' untranslated region (3' UTR) containing an AU-rich element, wherein said reporter gene is operably linked to said promoter region and said 3' UTR.
b. providing one or more protein kinase inhibitor(s) to be tested;
c. incubating the cells or a tissue created in step a. with said one or more protein kinase inhibitor(s) to be tested;
d. determining a normalizing effect of said one or more protein kinase inhibitor(s) on post-transcriptional regulation by determining a reporter activity, wherein a reduction in reporter activity indicates that said one or more protein kinase inhibitor(s) is/are suitable for targeted cancer therapy, wherein, preferably, the reduction is a reduction by at least 15 %, preferably by at least 20 %, more preferably by at least 25 %.

2. The method according to claim 1, wherein the targeted cancer therapy is a pan-cancer precision oncology therapy capable of treating a cancer regardless of the tissue type or subtype or molecular sub-type of the cancer including but not limited to solid tumors, hematological tumors, leukemias, lymphomas, organ-specific tumors such as breast, colon, prostate, liver, and metastatic tumors of any origin, including subtypes such as hormone positive, hormone negative, Microsatellite Instability high or low, and p53 mutant cancer.

3. The method according to claim 1, which is used as a universal single assay, that is applicable independently of a genetic lesion or type of molecular activity involved with regard to a cancerous disease of said patient.

4. The method according claim 1, wherein, in said targeted cancer therapy, a cancer-related gene is post-transcriptionally normalized by administering said protein kinase inhibitor.

5. The method according claim 1, wherein, in said targeted cancer therapy, a gene encoding a proinflammatory cytokine is post-transcriptionally normalized by administering said protein kinase inhibitor.

6. The method according to claims 4 or 5, wherein said administering of said protein kinase inhibitor results in the reduction of expression of a mRNA comprising an AU-rich element.

7. The method according to any of the foregoing claims, wherein said protein kinase inhibitor is selected from inhibitors of kinases of which a kinase activity is aberrant in cancer.

8. The method according to claim 1, wherein said method is to test whether a patient is likely to respond to a protein kinase inhibitor.

9. The method according to claim 1, wherein said method is to determine whether a protein kinase inhibitor is able to reduce reporter activity in a cancer cell of said patient comprising said expression vector, wherein a reduced reporter activity is an indicator that cancer cells of said patient will respond to said protein kinase inhibitor.

## Patentansprüche

1. In-vitro-Verfahren zum Identifizieren eines Proteinkinase-Inhibitors zum Normalisieren von post-transkriptioneller Regulierung, wobei der Inhibitor zur Verwendung in einer zielgerichteten Krebstherapie geeignet ist, wobei das Verfahren die folgenden Schritte umfasst:
a. Transfizieren von Krebszellen oder eines Gewebes eines Krebspatienten mit mindestens einem Expressionsvektor, umfassend:
i. eine Promoterregion, die einen nicht-induzierbaren, konstitutiv aktiven Promoter für ein Gen eines Ribosomenproteins umfasst, vorzugsweise einen Promoter, der einen modifizierten Promoter des humanen RPS30-Gens umfasst, der die Nukleinsäuresequenz von SEQ ID NO:3 (RPS30M1) oder SEQ ID NO:4 (RPS30M-trunkiert) aufweist,
ii. ein Reportergen; und
iii. eine 3'-untranslatierte Region (3'-UTR), die ein AU-reiches Element enthält, wobei das Reportergen operativ mit der Promoterregion und der 3'-UTR verbunden ist;
b. Bereitstellen von einem oder mehreren zu testenden Proteinkinase-Inhibitoren;
c. Inkubieren der in Schritt a) erzeugten Zellen oder eines Gewebes mit dem einen oder den mehreren zu testenden Proteinkinase-Inhibitoren;
d. Bestimmen eines normalisierenden Effekts des (der) einen oder mehreren Proteinkinase-Inhibitor(en) auf die post-transkriptionelle Regulierung durch Bestimmen einer Reporteraktivität, wobei eine Verringerung hinsichtlich Reporteraktivität darauf hinweist, dass der (die) eine oder mehreren Proteinkinase-Inhibitor(en) für die zielgerichtete Krebstherapie geeignet ist (sind), wobei bevorzugt die Verringerung eine Verringerung um mindestens 15 %, bevorzugt um mindestens 20 %, bevorzugter um mindestens 25 % beträgt.

2. Verfahren nach Anspruch 1, wobei die zielgerichtete Krebstherapie eine Präzisionsonkologie-Therapie für jeglichen Krebs ist, die in der Lage ist, einen Krebs zu behandeln, unabhängig vom Gewebetyp oder Subtyp oder molekularen Subtyp des Krebses, einschließlich, aber nicht beschränkt auf solide Tumoren, hämatologische Tumoren, Leukämien, Lymphome, organspezifische Tumoren, wie Brust-, Darm-, Prostata-, Leber- und metastasierte Tumoren jeglicher Herkunft, einschließlich Subtypen, wie Hormon-positiv, Hormon-negativ, mit hoher oder niedriger Mikrosatelliten-Instabilität sowie p53-mutierte Krebserkrankungen.

3. Verfahren nach Anspruch 1, das als universeller Einzelassay eingesetzt wird, der unabhängig von einer genetischen Läsion oder der Art der molekularen Aktivität, die im Zusammenhang mit einer Krebserkrankung des Patienten beteiligt ist, anwendbar ist.

4. Verfahren nach Anspruch 1, wobei bei der zielgerichteten Krebstherapie ein Krebsbezogenes Gen post-transkriptionell normalisiert wird, indem der Proteinkinase-Inhibitor verabreicht wird.

5. Verfahren nach Anspruch 1, wobei bei der zielgerichteten Krebstherapie ein Gen, welches für ein pro-inflammatorisches Zytokin kodiert, post-transkriptionell normalisiert wird, indem der Proteinkinase-Inhibitor verabreicht wird.

6. Verfahren nach den Ansprüchen 4 oder 5, wobei die Verabreichung des Proteinkinase-Inhibitors zur Verringerung der Expression einer mRNA führt, die ein AU-reiches Element umfasst.

7. Verfahren nach einem der vorangehenden Ansprüche, wobei der Proteinkinase-Inhibitor ausgewählt ist aus Inhibitoren von Kinasen, deren Kinase-Aktivität bei Krebs gestört ist.

8. Verfahren nach Anspruch 1, wobei das Verfahren darin besteht, zu testen, ob ein Patient wahrscheinlich auf einen Proteinkinase-Inhibitor anspricht.

9. Verfahren nach Anspruch 1, wobei das Verfahren darin besteht, zu bestimmen, ob ein Proteinkinase-Inhibitor in der Lage ist, eine Reporter-Aktivität in einer Krebszelle des Patienten, umfassend den Expressionsvektor, zu verringern, wobei eine verringerte Reporter-Aktivität ein Hinweis darauf ist, dass die Krebszellen des Patienten auf den Proteinkinase-Inhibitor ansprechen werden.

## Revendications

1. Procédé in vitro d'identification d'un inhibiteur de protéine kinase pour normaliser la régulation post-transcriptionnelle, lequel inhibiteur est adapté à être utilisé dans une thérapie ciblée contre le cancer, ledit procédé comprenant les étapes suivantes :
a. transfecter des cellules cancéreuses ou un tissu d'un patient atteint de cancer avec au moins un vecteur d'expression comprenant :
i. une région promotrice comprenant un promoteur de gène de protéine ribosomale constitutivement actif non inductible, de préférence un promoteur qui comprend un promoteur modifié du gène humain RPS30 qui a la séquence d'acide nucléique de SEQ ID NO:3 (RPS30M1) ou SEQ ID NO:4 (RPS30M-truncated),
ii. un gène rapporteur ; et
iii. une région non traduite 3' (3' UTR) contenant un élément riche en AU, dans laquelle ledit gène rapporteur est lié fonctionnellement à ladite région promotrice et ladite région non traduite 3' (3' UTR).
b. fournir un ou plusieurs inhibiteur(s) de protéine kinase à tester ;
c. incuber les cellules ou le tissu créé à l'étape a. avec ledit ou lesdits inhibiteur(s) de protéine kinase à tester ;
d. déterminer un effet de normalisation de ledit ou desdits inhibiteur(s) de protéine kinase sur la régulation post-transcriptionnelle en déterminant une activité du gène rapporteur, dans laquelle une réduction de l'activité du gène rapporteur indique que ledit ou lesdits inhibiteur(s) de protéine kinase est/sont adapté(s) à une thérapie ciblée contre le cancer, dans laquelle, de préférence, la 1éductio nest une réduction d'au moins 15 %, de préférence d'au moins 20 %, plus préférablement d'au moins 25 %.

2. Procédé selon la revendication 1, dans lequel la thérapie ciblée contre le cancer est une thérapie oncologique de précision pan-cancer capable de traiter un cancer indépendamment du type de tissu ou du sous-type ou du sous-type moléculaire du cancer, y compris mais sans s'y limiter, des tumeurs solides, des tumeurs hématologiques, des leucémies, des lymphomes, des tumeurs spécifiques d'organe telles que le sein, le côlon, la prostate, le foie, et des tumeurs métastatiques de toute origine, y compris des sous-types tels que hormone positif, hormone négatif, instabilité des microsatellites élevée ou faible, et cancer mutant p53.

3. Procédé selon la revendication 1, qui est utilisé comme un test universel unique, qui est applicable indépendamment d'une lésion génétique ou du type d'activité moléculaire impliquée concernant une maladie cancéreuse dudit patient.

4. Procédé selon la revendication 1, dans lequel, dans ladite thérapie ciblée contre le cancer, un gène lié au cancer est normalisé post-transcriptionnellement par administration dudit inhibiteur de protéine kinase.

5. Procédé selon la revendication 1, dans lequel, dans ladite thérapie ciblée contre le cancer, un gène codant une cytokine pro-inflammatoire est normalisé post-transcriptionnellement par administration dudit inhibiteur de protéine kinase.

6. Procédé selon l'une quelconque des revendications 4 ou 5, dans lequel ladite administration dudit inhibiteur de protéine kinase entraîne la réduction de l'expression d'un ARNm comprenant un élément riche en AU.

7. Procédé selon l'une quelconque des revendications précédentes, dans lequel ledit inhibiteur de protéine kinase est sélectionné parmi des inhibiteurs de kinases dont une activité kinase est aberrante dans le cancer.

8. Procédé selon la revendication 1, dans lequel ledit procédé sert à tester si un patient est susceptible de répondre à un inhibiteur de protéine kinase.

9. Procédé selon la revendication 1, dans lequel ledit procédé sert à déterminer si un inhibiteur de protéine kinase est capable de réduire l'activité du gène rapporteur dans une cellule cancéreuse dudit patient comprenant ledit vecteur d'expression, dans lequel une activité du gène rapporteur réduite est un indicateur que les cellules cancéreuses dudit patient répondront audit inhibiteur de protéine kinase.
